# EUROPEAN PATENT APPLICATION

(11) **EP 4 528 752 A1**
(43) Date of publication of application: **26.03.2025**
(21) Application number: 23807935.4
(22) Date of filing: 18.05.2023
(51) Int. Cl.: G16H 50/80, G16H 50/20, G16H 10/40, G16B 15/00

(54) **METHOD FOR CONTROLLING DISPLAYING OF MOLECULAR DIAGNOSTIC RESULTS AND COMPUTER DEVICE FOR PERFORMING SAME**

(30) Priority: 19.05.2022 KR 20220061235
(71) Applicant: Seegene, Inc., Seoul 05548 (KR)
(72) Inventor: KIM, Se Ryun, Hwaseong-si Gyeonggi-do 18430 (KR); OH, Won Jun, Yongin-si Gyeonggi-do 16822 (KR)
(74) Representative: Viering, Jentschura & Partner mbB Patent- und Rechtsanwälte
(86) International application number: PCT/KR2023/006802
(87) International publication number: WO 2023/224416

(57) **Abstract**

According to an embodiment, disclosed is a method for controlling display of molecular diagnostic results performed by a computer device. The method may include: obtaining diagnostic results performed on a plurality of examinees in order to detect a respiratory virus or a human papilloma virus (HPV); wherein the respiratory virus comprises at least two types of respiratory viruses as a target virus and the HPV comprises at least two types of HPV subtypes as a target virus; calculating, from the diagnostic results, at least one of: (a) a first cycle threshold (C_{T}) distribution range obtained from a specific target virus in a single detection case where only the specific target virus among a plurality of target viruses is detected, and a second C_{T} distribution range obtained from the specific target virus in a concurrent detection case where one or more other target virus is detected together with the specific target virus; and (b) a count of a first examinees corresponding to the single detection case among the plurality of examinees, and a count of a second examinees corresponding to the concurrent detection case among the plurality of examinees; and controlling display of at least one of the C_{T} distribution range and the count of examinees on a single screen of the computer device or a user terminal.

## Description

### TECHNICAL FIELD

The present disclosure relates to a method for controlling display of molecular diagnostic results and a computer apparatus for performing the same.

### BACKGROUND ART

In order to support users to obtain meaningful insights from multiple molecular diagnostic test results, a process is required to control that the test results are displayed intuitively from the user's perspective. Here, the user may be hospitals who are interested in molecular diagnostic results, research and development companies of molecular diagnostic kits, academic institutions, research institutes, or government agencies such as the Korea Disease Control and Prevention Agency.

There are various ways to display the test results. For example, there is a method of counting and displaying the count of positive cases for a specific pathogen among the test results. However, these conventional technologies only list fragmentary information about the test results, and there is a limitation that it is difficult for the user to obtain meaningful insights from them.

In addition, there is virtually no method to display the test results of patients concurrently infected (co-infected) with multiple pathogens, such as patients co-infected with influenza and COVID-19. According to the conventional technologies, when obtaining information on such concurrently infected patients, the users themselves are required to classify cases of co-infected patients from the test results and compare and analyze them. Accordingly, there is a problem of low user convenience.

### DETAILED DESCRIPTION OF INVENTION

### TECHNICAL PROBLEM

A problem to be solved by an embodiment of the present disclosure is to provide a control method that displays values of items meaningful to the user in a mutually comparable manner, allowing the user to more conveniently obtain meaningful insights from the diagnostic results.

However, problems to be solved by the present disclosure are not limited to the foregoing, and other unmentioned objects would be apparent to one of ordinary skill in the art from the following description.

### TECHNICAL SOLUTION

Disclosed is a method for controlling display of molecular diagnostic results performed by a computer device according to an embodiment of the present disclosure. The method includes: obtaining diagnostic results performed on a plurality of examinees in order to detect a respiratory virus or a human papilloma virus (HPV); wherein the respiratory virus comprises at least two types of respiratory viruses as a target virus and the HPV comprises at least two types of HPV subtypes as a target virus; calculating, from the diagnostic results, at least one of: (a) a first cycle threshold (C_{T}) distribution range obtained from a specific target virus in a single detection case where only the specific target virus among a plurality of target viruses is detected, and a second C_{T} distribution range obtained from the specific target virus in a concurrent detection case where one or more other target virus is detected together with the specific target virus; and (b) a count of a first examinees corresponding to the single detection case among the plurality of examinees, and a count of a second examinees corresponding to the concurrent detection case among the plurality of examinees; and controlling display of at least one of the C_{T} distribution range and the count of examinees on a single screen of the computer device or a user terminal.

In addition, the method may further include: filtering the diagnostic results by applying a predetermined first condition for at least one of a period, a season, a region, an age and a target virus, and a molecular diagnostic product, wherein the diagnostic results in the calculating, is the filtered diagnostic results.

In addition, wherein the controlling the display of the computer device or the user terminal may include: providing a region corresponding to the specific target virus on the single screen, and controlling that the first C_{T} distribution range and the second C_{T} distribution range are displayed in the region.

In addition, wherein the controlling the display of the computer device or the user terminal may include: controlling that at least one of a median value, a first quartile, a third quartile, an average value and a mode value is displayed in each of the first C_{T} distribution range and the second C_{T} distribution range.

In addition, wherein the controlling the display of the computer device or the user terminal may include: controlling that at least one of an interquartile range (IQR) indicating a difference between the first quartile and the third quartile, a minimum value calculated based on the first quartile and the IQR, and a maximum value calculated based on the third quartile and the IQR is further displayed in each of the first C_{T} distribution range and the second C_{T} distribution range.

In addition, wherein the controlling the display of the computer device or the user terminal may include: controlling that each of the first C_{T} distribution range and the second C_{T} distribution range is displayed in a box plot method.

In addition, wherein the controlling the display of the computer device or the user terminal may include: providing a region corresponding to the specific target virus on the single screen, and controlling that the count of the first examinees and the count of the second examinees are displayed in the region.

In addition, wherein the controlling the display of the computer device or the user terminal may include: adding an identifiable mark for one or more target viruses of which a difference between the count of the first examinees and the count of the second examinees or a difference between a representative value of the first C_{T} distribution range and a representative value of the second C_{T} distribution range is not less than a predetermined reference value among the plurality of target viruses.

In addition, wherein the controlling the display of the computer device or the user terminal may include: for at least one target virus in which the count of the second examinees is not less than a predetermined reference value than the count of the first examinees among the plurality of target viruses, adding an identifiable mark when a representative value of the C_{T} distribution range is not less than the representative value of the C_{T} distribution range in the single detection case.

In addition, wherein the method may further include controlling display of information about one or more other target viruses detected together with the specific target virus in the concurrent detection case of the computer device or the user terminal.

In addition, wherein the information about the one or more other target viruses may include: (a) a name of each of the one or more other target viruses detected together with the specific target virus; and (b) a count of examinees and/or a C_{T} distribution range in the concurrent detection case in which each of the one or more other target viruses are detected together with the specific target virus.

In addition, wherein the information about the one or more other target viruses may include: (a) names of a plurality of combinations for the one or more other target viruses detected together with the specific target virus, and (b) a count of examinees and/or a C_{T} distribution range in a concurrent detection case in which each of the plurality of combinations are detected together with the specific target virus.

In addition, wherein the controlling the display of the information about the one or more other target viruses may be performed corresponding to selection of a screen region displaying the second C_{T} distribution range or the count of the second examinees.

In addition, wherein the method may further include: controlling display of information about the concurrent detection case of the specific target virus and information about the concurrent detection case of additional specific target virus on a single screen of the computer device or the user terminal.

In addition, wherein the method may further include: when any one of the specific target viruses is selected among the specific target viruses, controlling display of information about a concurrent detection case of the selected any one specific target virus of the computer device or the user terminal.

In addition, wherein the at least two types of the respiratory viruses may be selected from the group consisting of influenza virus, respiratory syncytial virus (RSV), adenovirus, enterovirus, parainfluenza virus (PIV), metapneumovirus (MPV), bocavirus, rhinovirus, and coronavirus.

In addition, wherein the at least two types of HPV subtypes may be selected from the group consisting of HPV-16, -18, -26, -30, -31, -34, -35, - 39, -45, -51, -52, -53, -56, -58, -59, -61, -66, -67, -68, -69, -70, and -73 belonging to a high-risk (HR) group.

In addition, wherein the diagnostic results may be characterized in that they are diagnostic results generated by using the identical qPCR device and the identical same extraction method.

In addition, wherein the diagnostic results may be characterized in that they are diagnostic results generated by using the identical qPCR device, the identical extraction method, the identical target signaling mechanism, and the identical polymerase mastermix.

Disclosed is a computer program stored on a computer-readable recording medium according to an embodiment of the present disclosure. The computer program is programmed to perform each of steps included in the method.

Disclosed is a computer-readable recording medium on which a computer program is stored according to an embodiment of the present disclosure. The computer program is programmed to perform each of steps included in the method.

Disclosed is a computer device according to an embodiment of the present disclosure. The computer device includes: a memory configured to store at least one instruction; and a processor configured to execute the one or more instructions stored in the memory, wherein the instructions, when executed by the processor, cause the processor to: obtaining, by the processor, diagnostic results performed on a plurality of examinees in order to detect a respiratory virus or a human papilloma virus (HPV); wherein the respiratory virus comprises at least two types of respiratory viruses as a target virus and the HPV comprises at least two types of HPV subtypes as a target virus; calculating, by the processor, from the diagnostic results, at least one of: (a) a first cycle threshold (C_{T}) distribution range obtained from a specific target virus in a single detection case where only the specific target virus among a plurality of target viruses is detected, and a second C_{T} distribution range obtained from the specific target virus in a concurrent detection case where one or more other target virus is detected together with the specific target virus; and (b) a count of a first examinees corresponding to the single detection case among the plurality of examinees, and a count of a second examinees corresponding to the concurrent detection case among the plurality of examinees; and controlling, by the processor, display of at least one of the C_{T} distribution range and the count of examinees on a single screen of the computer device or a user terminal.

### ADVANTAGEOUS EFFECTS

The computer device according to an embodiment of the present disclosure may support the user to derive a meaningful insight through an intuitive comparison of the values, by controlling display of a cycle threshold (C_{T}) distribution range and/or a count of corresponding examinees in each single detection case and each concurrent detection case both for a specific target analyte on a single screen.

The effects of the present disclosure are not limited to the foregoing, and should be understood to include all effects that can be inferred from the configuration of the present disclosure described in the detailed description or claims of the present disclosure.

### BRIEF DESCRIPTION OF THE DRAWINGS

FIG. 1 is a block diagram illustrating a configuration of a diagnostic result providing system according to an embodiment.
FIG. 2 is a block diagram schematically illustrating a configuration of the computer device according to an embodiment.
FIG. 3 is a diagram illustrating modularized software implemented by the computer device illustrated in FIG. 2.
FIG. 4 is a diagram exemplarily illustrating that the first C_{T} distribution range and the second C_{T} distribution range for a specific target virus are displayed on a single screen according to the first embodiment.
FIG. 5 is a diagram exemplarily illustrating that the count of the first examinees and the count of the second examinees for the specific target virus are displayed on a single screen according to the second embodiment.
FIG. 6 is a diagram exemplarily illustrating that information about the concurrent detection case of the specific target virus and information about the concurrent detection case of an additional specific target virus are displayed on a single screen according to the fourth embodiment.
FIG. 7 is a flowchart illustrating a method for controlling display of molecular diagnostic results performed by the computer device according to an embodiment.
FIG. 8 is a diagram exemplarily illustrating that information about one or more other target viruses detected together with the specific target virus in the concurrent detection case are displayed on a single screen according to the third embodiment.

### MODE FOR CARRYING OUT THE INVENTION

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

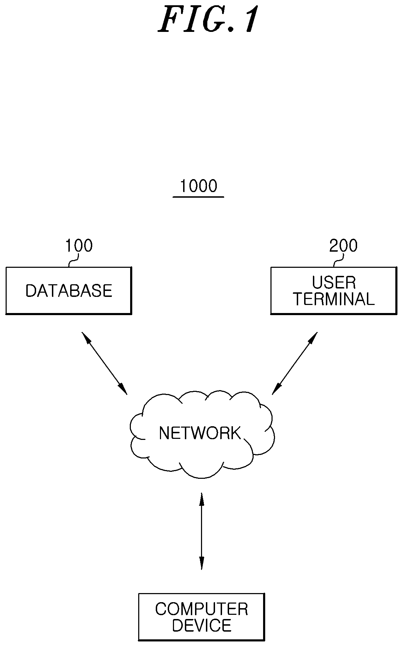

Prior to describing FIG. 1, terms used herein are described.

As used herein, the term "diagnostic result" refers to an in vitro diagnostic result performed using a material derived from a human body, and refers to a result obtained based on a signal generation reaction. As used herein, the term "signal generation reaction" refers to a reaction that generates a signal dependent on the properties of a target analyte in a sample, e.g., activity, amount, or presence (or absence), specifically generates a signal dependent on the presence (or absence). This signal generation reaction includes a biological reaction and a chemical reaction. Among them, the biological reaction includes a genetic analysis process such as polymerase chain reaction (PCR), real-time PCR, real-time isothermal amplification reaction, and microarray analysis, an immunological analysis process, and a bacterial growth analysis. In addition, the chemical reaction includes a process of analyzing the production, change, or destruction of a chemical substance.

According to an embodiment, the signal generation reaction may be the genetic analysis process, or may be a nucleic acid amplification reaction such as the PCR or the isothermal amplification reaction, an enzymatic reaction, or microbial growth. In the amplification reaction, amplification of a target analyte (e.g., a nucleic acid molecule) may or may not be accompanied. More specifically, the amplification reaction may mean an amplification reaction of a signal accompanied by amplification of the target analyte. As a representative example of the nucleic acid amplification reaction, in the PCR, a repeated cycle process of denaturation of double-stranded DNA, annealing of an oligonucleotide primer to a DNA template, and primer extension by DNA polymerase is performed (Mullis et al., U.S. Pat. Nos. 4, 683, 195, 4, 683, 202, and 4, 800, 159; Saiki et al., Science 230: 1350-1354 (1985)). As other methods for the amplifying the nucleic acid, various methods such as Ligase Chain Reaction (LCR), Strand Displacement Amplification (SDA), Nucleic Acid Sequence-Based Amplification (NASBA), Transcription Mediated Amplification (TMA), Recombinase Polymerase Amplification (RPA), Loop-mediated isothermal amplification (LAMP), and Rolling-Circle Amplification (RCA) have been proposed.

According to an embodiment, the nucleic acid amplification reaction may be used in a nucleic acid amplification test (NAAT). The NAAT may be used in a variety of technical fields for detecting a target analyte by using the nucleic acid amplification reaction. In an embodiment, the NAAT may be performed on various objects such as soil, water, and plants, and by detecting whether nucleic acid molecules extracted from a pathogen (e.g., virus) are included in such objects, it is possible to test for soil contamination, water contamination, or pathogen infection of animals and plants.

In addition, the term "diagnostic result" may refer to, for example, a diagnostic result performed on an examinee, and may refer to result data such as whether a target analyte has been detected in a sample obtained from the corresponding examinee (e.g., positive/negative information), or a signal value or a measurement value (e.g., a cycle threshold (CT) value) derived in a detection process. According to an embodiment, the diagnostic result may include data obtained by processing such result data. In an embodiment, the diagnostic result may include a molecular diagnostic result, and may include, for example, PCR result data. In an embodiment, the diagnostic result may be an NAAT result obtained by using an NAAT panel.

In addition, the term "target analyte" may refer to various substances (e.g., biological substances and non-biological substances). Specifically, the target analyte may include a biological substance, more specifically at least one of nucleic acid molecules (e.g., DNA and RNA), proteins, peptides, carbohydrates, lipids, amino acids, biological compounds, hormones, antibodies, antigens, metabolites, and cells.

According to an embodiment, the target nucleic acid molecule refers to a nucleotide molecule in an organism to be detected. The target nucleic acid molecule is generally designated by a specific name and includes the entire genome and all nucleotide molecules that make up the genome (e.g., genes, pseudogenes, non-coding sequence molecules, non-reading regions, and some regions of the genome). The target nucleic acid molecule includes, for example, nucleic acid of an organism. Here, the organism means an organism that belongs to a biological classification system, such as a kingdom, division, class, order, family, genus, species, subspecies, forma, variety, subtype, genotype, sirotype, strain, isolate, or cultivar. The organism may include, for example, prokaryotic cells, eukaryotic cells, viruses, or viroids. In an embodiment in which a target analyte (or target nucleic acid molecule) includes the nucleic acid molecule of the virus to be detected, the target analyte (or target nucleic acid molecule) may be used interchangeably with the target virus.

According to an embodiment, the virus may mean at least one of respiratory virus, human papilloma virus (HPV), a virus that causes gastrointestinal disease, Middle East respiratory syndrome-related coronavirus (MERS-CoV), Dengue virus, Herpes simplex virus (HSV), Human herpes virus (HHV), Epstein-Barr virus (EMV), Varicella zoster virus (VZV), Cytomegalovirus (CMV), HIV, hepatitis virus, and poliovirus.

The respiratory virus may include at least one of influenza virus (e.g., influenza A virus and influenza B virus), RSV (respiratory syncytial virus) (e.g., RSV A and RSV B), Adenovirus (AdV), enterovirus, PIV (parainfluenza virus) (e.g., PIV 1, PIV 2, PIV 3, and PIV 4), MPV (metapneumovirus), bocavirus, rhinovirus (e.g., HRV (human Rhinovirus)), coronavirus (e.g., CoV NL63, CoV 229E, CoV OC43, CoV HKU1, SARS-CoV (severe acute respiratory syndrome coronavirus 2), MERS-CoV, SARS-CoV-2), Mycoplasma pneumoniae, Chlamydophila pneumoniae, Legionella pneumoniae, Haemophilus influenza, Streptococcus pneumoniae, Bordetella pertussis and Bordetella parapertussis. According to an embodiment, the respiratory virus may include at least two types of respiratory viruses. Here, the term "type" refers to a classification used in the biological classification system, and may be, for example, the genus, the species, the subspecies, the subtype, or the like.

The HPV may include at least one of the known subtypes of HPV. According to an embodiment, the HPV may include at least one of HPV-16, -18, -26, -30, - 31, -34, -35, -39, -45, -51, -52, -53, -56, -58, -59, -61, -66, -67, -68, -69, -70, and -73 belonging to the high-risk group (HR) for cervical cancer. Alternatively, according to an embodiment, the HPV may further include at least one of HPV-2, -3, -6, -7, -10, -13, -32, -40, -42, -43, -44, -55, -54 and -57 belonging to a low-risk group (LR) for cervical cancer. According to an embodiment, the HPV may include at least two types of HPV subtype.

The virus causing the gastrointestinal disease may include at least one of norovirus, rotavirus, adenovirus, astrovirus and sapovirus.

In addition, the term "sample" may mean a biological sample (e.g., cells, tissues, and body fluids) and a non-biological sample (e.g., food, water, and soil). The biological sample may include at least one of, for example, virus, bacteria, tissue, cells, blood (including whole blood, plasma, and serum), lymph, bone marrow, saliva, sputum, swab, aspiration, milk, urine, stool, eye fluid, semen, brain extract, spinal fluid, joint fluid, thymus fluid, bronchial lavage fluid, ascites, and amniotic fluid. These samples may or may not include the target analyte described above.

On the other hand, when the target analyte described above is a nucleic acid molecule or includes a nucleic acid molecule, a nucleic acid extraction process known in the art may be performed on the sample assumed to include the target analyte (see Sambrook, J. et al., Molecular Cloning. A Laboratory Manual, 3rd ed. Cold Spring Harbor Press(2001)). The nucleic acid extraction process may vary depending on the type of sample. In addition, when the extracted nucleic acid is RNA, a reverse transcription process for synthesizing cDNA may be additionally performed (see Sambrook, J. et al., Molecular Cloning. A Laboratory Manual, 3rd ed. Cold Spring Harbor Press(2001)).

In addition, the term "single detection case" refers to a case in which only one specific target analyte among a plurality of target analytes to be detected in one sample is detected. For example, the single detection case may refer to a case of a single infection in which only a specific target pathogen among a plurality of target pathogens is determined to be positive, and according to an embodiment, the case may include a case in which the remaining target pathogens other than the corresponding target pathogen are determined to be negative and/or the determination result of the remaining target pathogens is not obtained. As another example, the single detection case may refer to a single detection case in which only specific target nucleic acid molecules among the plurality of target nucleic acid molecules is detected, and is not limited to the above-described embodiment.

In addition, the term "concurrent detection case" refers to a case in which one or more other target analytes are detected together with a specific target analyte among a plurality of target analytes to be detected in one sample pool. For example, the concurrent detection case may refer to a case of co-infection in which one or more other target pathogens are detected together with a specific target pathogen among a plurality of target pathogens. According to an embodiment, the concurrent detection case may refer to a case of a double infection in which an examinee already infected with a specific target pathogen is infected with another type of target pathogen. In addition, as another example, the concurrent detection case may refer to a case in which one or more other dielectric materials are detected together with a specific dielectric material among the plurality of nucleic acid molecules, and is not limited to the above-described embodiment.

Hereinafter, various embodiments of the present disclosure will be described with reference to the drawings.

FIG. 1 is a block diagram illustrating a configuration of a diagnostic result providing system 1000 according to an embodiment.

Referring to FIG. 1, the diagnostic result providing system 1000 may include at least one of a database 100, a user terminal 200, and a computer device 300.

The database 100 may store diagnostic results performed on a plurality of examinees. Here, each diagnostic result may be a diagnostic result performed at least one of a plurality of target analytes as a target. In an example, the diagnostic result may be performed by targeting only a first target analyte (e.g., AdV) among the plurality of target analytes, and in another one example, may be performed by targeting both the first target analyte and a second target analyte (e.g., HRV), and in another one example, may be performed by targeting all of the first target analyte, the second target analyte, and a third target analyte (e.g., PIV2). In an embodiment, each of the diagnostic results may be a diagnostic result performed on different examinee, but is not limited thereto. For example, when the target analytes are different, the diagnostic result may be a diagnostic result performed on the same examinees.

In an embodiment, the diagnostic result may be performed on an examinee to detect the respiratory virus or the HPV. Here, detecting the respiratory virus or the HPV may mean detecting whether a target nucleic acid molecule separated from the corresponding respiratory virus or HPV is present in a sample taken from the examinee.

In an embodiment, the diagnostic result may be a diagnostic result performed to detect at least two types of respiratory viruses as a target virus to be detected, or may be a diagnostic result performed to detect at least two types of HPV subtypes. For example, each diagnostic result may be a multiplex diagnostic result for concurrently detecting a plurality of target viruses including influenza virus, RSV, coronavirus, MPV and rhinovirus belonging to the respiratory viruses. According to an embodiment, some of the diagnostic results may be singleplex diagnostic results for detecting only any one of these target viruses.

In an embodiment, the diagnostic results may be diagnostic results generated by using the identical quantitative real time PCR (qPCR) device and the identical extraction method. Here, the identical may comprehensively mean a case in which: the objects are the same; the types of the objects are the same; or, the objects or the types of the objects belong to a predetermined category. As a specific example, the identical device may mean that the type of the corresponding devices belong to the same category, and the identical method may mean that mechanisms or algorithms defining the corresponding method belong to the same category. The qPCR device refers to at least one of device used to obtain an qPCR result, and specific examples thereof include a nucleic acid extraction device (e.g., SEEPREP32), a setup device for nucleic acid amplification (e.g., NIMBUS, STARlet), a nucleic acid amplification device (e.g., CFX96), and a device for analyzing amplified data (e.g., a computer device in which a data analysis program is installed). In addition, the extraction method refers to a method used in at least one process of sampling, lysis, binding, washing, and elution for the nucleic acid extraction, and specific examples thereof include a bead transfer method, a liquid transfer method, and the like.

In one embodiment, the diagnostic results may be diagnostic results generated by using the identical qPCR device, the identical extraction method, the identical target signaling mechanism, and the identical polymerase mastermix. Here, the target signaling mechanism refers to a signaling mechanism used to distinguish amplification results of different targets in the qPCR process, and may include, for example, a TaqMan probe method, a Tagging Oligonucleotide Cleavage and Extension (TOCE) method, or the like. In addition, the polymerase mastermix may be understood as a comprehensive term that includes (a) a reaction environment of the nucleic acid amplification reaction or (b) a reaction medium added to make the reaction environment. Here, the reaction medium may be one or more materials selected from the group consisting of a pH-related material (e.g., buffer), an ion intensity-related material (e.g., Mg²⁺, K⁺), an enzyme (e.g., a nuclease, a nucleic acid polymerase, a linking enzyme, a modifying enzyme, etc.) and an enzyme stabilization-related material (e.g., sugar).

In an embodiment, the diagnostic result may include at least one of examinee data, target analyte data, and positive/negative determination data.

Here, the examinee data refers to data about an examinee who has undergone the diagnostic test. In an embodiment, the examinee data may include an examinee identifier (e.g., a resident registration number, an ID, etc.), and may further include personal data such as nationality, residential area, age, gender, occupation, and the like of the examinee.

In addition, the target analyte data refers to data about at least one target analyte targeted by the diagnostic result. In an embodiment, the target analyte data may include a target analyte identifier (e.g., a unique name, a management number, an ID, etc.) of each of one or more target analytes to be targeted, and may further include data about each substance type (e.g., nucleic acid molecule, protein, etc.) and, when the target analyte is a pathogen, types of infectious diseases that may occur due to the pathogen (e.g., Respiratory, Gastrointestinal Tract, etc.). In an embodiment, the target analyte data may further include a group identifier for a group of target analytes, and may further include, for example, a group ID for identifying whether the target viruses belong to the respiratory virus or the HPV.

In addition, the positive/negative determination data may include positive/negative data (e.g., the presence or the absence of the target analyte) indicating whether the target analyte is detected or not, and may further include one or more signal values or measurement values used as a criterion for determining positive or negative. In an embodiment, the signal value may include (a) a magnitude value of a fluorescence signal obtained from a nucleic acid amplification device, such as relative fluorescence unit (RFU), or the like, or (b) a value obtained by processing the magnitude value in a predetermined manner. In addition, the measurement value may include a time (e.g., a cycle value) when the signal value corresponds to a predetermined threshold, such as a cycle threshold (C_{T}), but is not limited thereto, and may include various other values that can be used as a relative measurement of the concentration of the target analyte.

In an embodiment, the positive/negative determination data may include the positive/negative data and the C_{T} value of each of the plurality of target viruses when the diagnostic result is the multiplex diagnostic result, and may include the positive/negative data and the C_{T} value of one target virus when the diagnostic result is the singleplex diagnostic result.

In an embodiment, the diagnostic result may further include data about at least one of the qPCR device, the extraction method, the target signaling mechanism, and the polymerase mastermix described above. For example, each diagnostic result may be a diagnostic result in which at least one of the qPCR device, the extraction method, the target signaling mechanism, and the polymerase mastermix is different from each other, and may further include data about the qPCR device, the extraction method, the target signaling mechanism, and the polymerase mastermix used in each nucleic acid amplification process. Such data may be used in a subsequent process for filtering the diagnostic result, and the filtering will be described later.

In an embodiment, in the database 100, diagnostic results including the examinee data, the target analyte data, and the positive/negative determination data may be stored and managed as a dataset. In addition, the above-described data, for example, the examinee identifier, the target analyte identifier, and the positive/negative result value may be used to query or search for the diagnostic result in the database 100 as metadata of each diagnostic result.

In an embodiment, the diagnostic result may be periodically collected in the database 100 by at least one of a separate management server (not shown), a medical institution server (not shown), and the computer device 300. For an example, a diagnostic result may be collected by (a) a computer in which a viewer program analyzing a nucleic acid amplification result generated by the nucleic acid amplification device and providing positive/negative data is executed or (b) an internal server or the computer device 300 connected the computer. For another example, the diagnostic result may be collected from an external server (e.g., the medical institution server) that stores and manages a plurality of previously obtained diagnostic results for a plurality of examinees.

The user terminal 200 may be a device such as a computer used by a user. In an embodiment, the user terminal 200 may be implemented as various types of handheld-based wireless communication devices, such as a mobile phone, a smart phone, a personal digital assistant (PDA), a portable multimedia player (PMP), a tablet PC, and the like. Alternatively, the user terminal 200 may include various types of wired/wireless communication devices, such as a desktop PC, a tablet PC, a laptop PC, and the like, which are connected to an external server and have a foundation for installing and executing applications.

The computer device 300 may be connected to at least one of the database 100, the one or more user terminals 200, and other devices (e.g., a terminal, a server) through a network or electrically. Here, the network may be configured through various communication networks such as wired and wireless networks, and for example, may be configured as various communication networks such as a Local Area Network (LAN), a Metropolitan Area Network (MAN), a Wide Area Network (WAN), and the like.

The computer device 300 may obtain a plurality of diagnostic results from the database 100. For example, the computer device 300 may access the database 100 and search the plurality of diagnostic results stored in the database 100. Although FIG. 1 illustrates that the computer device 300 and the database 100 are implemented separately, this is merely an example. For example, the computer device 300 may be implemented to include the database 100.

The computer device 300 may be implemented to provide information on a predetermined item in each of the single detection case and the concurrent detection case for a specific target analyte from the plurality of obtained diagnostic results. In an embodiment, the predetermined item may include a count of examinees corresponding to each case and the like, and it will be described in detail later.

In an embodiment, the computer device 300 may be implemented as a computer operating through a computer program for fulfilling the functions described in the present specification. For example, the computer device 300 may be implemented as a computer used by a user. Here, the user may be a public office such as a hospital, a research and development company of a molecular diagnostic kit, a school, a research institute, or the Korea Centers for Disease Control and Prevention, which is interested in a molecular diagnostic result. In addition, the functions described herein may be implemented in software and mounted in the computer device 300. In addition, the computer device 300 may receive the plurality of diagnostic results from the database 100 based on the authority assigned to the user account, generate information on a predetermined item in each of the single detection case and the concurrent detection case for a specific target analyte from the plurality of received diagnostic results, and control display of the generated information on a single screen of the computer device 300.

In another embodiment, the computer device 300 may be implemented as a server operating through a computer program for fulfilling the functions described in the present specification. For example, the computer device 300 may receive a request for providing information on the predetermined item from the user terminal 200, receive the plurality of diagnostic results from the database 100 according to the received request, and generate information on the predetermined item in each of the single detection case and the concurrent detection case for a specific target analyte from the plurality of received diagnostic results. In addition, the computer device 300 may transmit the generated information to the user terminal 200 and control display of the generated information on a single screen of the user terminal 200.

Meanwhile, the diagnostic result providing system 1000 may further include other components in addition to the components shown in FIG. 1. Alternatively, one or more of the components illustrated in FIG. 1 may be omitted.

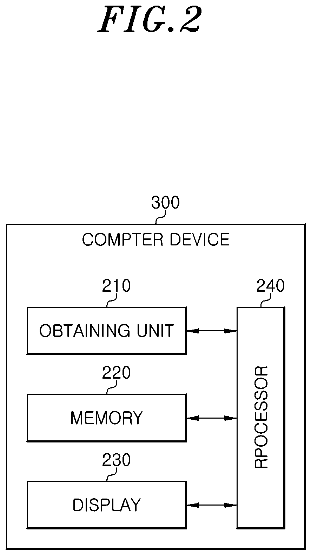

FIG. 2 is a block diagram schematically illustrating a configuration of the computer device 300 according to an embodiment.

Referring to FIG. 2, the computer device 300 may include an obtaining unit 210, a memory 220, a display 230, and a processor 240.

The obtaining unit 210 may obtain a diagnostic result performed on a plurality of examinees targeting at least one of a plurality of target analytes. As described above, the diagnostic result may include diagnostic results performed on the plurality of examinees targeting a specific target analyte among the plurality of target analytes, and diagnostic results performed on the plurality of examinees targeting one or more other target analytes together with the corresponding target analyte.

In an embodiment, the diagnostic result may include the diagnostic result performed on the plurality of examinees to detect the respiratory virus or HPV. As described above, the respiratory virus may include at least two types of respiratory viruses as a target virus, and the HPV may include at least two types of HPV subtype as the target virus.

In an embodiment, the obtaining unit 210 may obtain the diagnostic result from the database 100. For example, the obtaining unit 210 may obtain, from the database 100, a diagnostic result performed to detect the respiratory virus or HPV for a predetermined period. For another example, the diagnostic result performed targeting at least two types of respiratory viruses may be obtained from the database 100.

In an embodiment, the obtaining unit 210 may include a transceiver (not shown), and may receive the diagnostic result from the database 100 by using the transceiver. In another embodiment, the obtaining unit 210 may include a data input port (not shown), and may read the diagnostic result stored in a corresponding device from a storage device (e.g., a universal serial bus (USB), the memory 220, or the like) electrically connected to the computer device 300 by using the data input port, but is not limited to the above-described embodiment. In addition, the operation of the obtaining unit 210 may be controlled by the processor 240, and may be performed by executing an instruction(s) stored in the memory 220 by the processor 240.

The memory 220 may store at least one instruction or data to be executed by the processor 240 to be described below. In an embodiment, the memory 220 may include at least one of a read only memory (ROM) and a random access memory (RAM).

The display 230 may display an image, a graph, a table, a text, or the like obtained as at least one instruction is executed by the processor 240. For example, the display 230 may display information on the predetermined item in each of the single detection case and the concurrent detection case for a specific target analyte generated by the processor 240 as an image or text. Meanwhile, the display in the disclosure may be understood as a meaning including display or output of information, and for example, a certain value displayed on the screen may mean that data indicating the corresponding value is visually displayed or output in a predetermined data format.

The display 230 may comprehensively mean a data output device for displaying data. For example, the display 230 may be implemented as a liquid crystal display, a thin film transistor-liquid crystal display, an organic light-emitting diode, a flexible display, a three-dimensional (3D) display, or the like.

The processor 240 may control the overall operation of the computer device 300, and may perform a series of operations of controlling a display of the diagnostic result. For example, the processor 240 may perform embodiments described throughout the specification or may control the components of the computer device 300 such that the embodiments are performed. The processor 240 may refer to a central processing unit (CPU), a graphics processing unit (GPU), a micro controller unit (MCU), or a dedicated processor on which methods according to embodiments are performed.

In addition, it may be understood by those of ordinary skill in the art that other general-purpose components other than the components illustrated in FIG. 2 may be further included in the computer device 300. For example, the computer device 300 may further include a bus line used for transmission of data between the components, various input/output devices, an interface therefor, and the like. In addition, one or more of the components illustrated in FIG. 2 may be omitted.

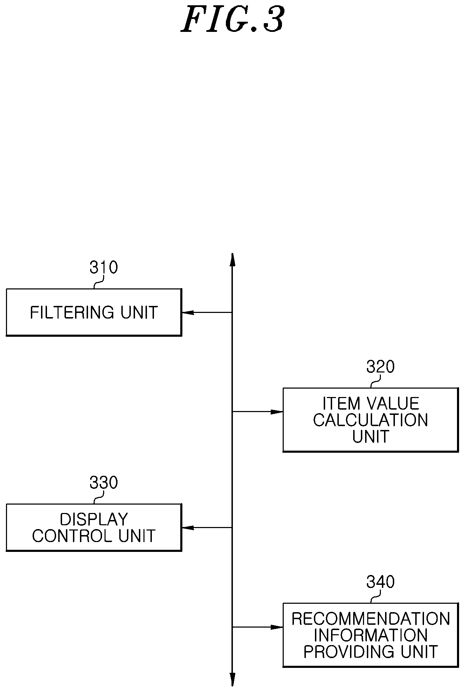

The processor 240 may provide various functions by executing at least one instruction stored in the memory 220. Various functions provided by the processor 240 will be described with reference to FIG. 3.

FIG. 3 is a diagram illustrating modularized software implemented by the computer device 300 illustrated in FIG. 2. Referring to FIG. 3, software implemented as the processor 240, which is hardware, executes at least one instruction stored in the memory 220 may be modularized into at least one of a filtering unit 310, an item value calculation unit 320, a display control unit 330, and a recommendation information providing unit 340. For example, each of the filtering unit 310, the item value calculation unit 320, the display control unit 330, and the recommendation information providing unit 340 may be implemented as a computer program, and instructions and data for execution thereof may be stored in the memory 220 and executed by the processor 240, but are not limited thereto.

The filtering unit 310 may be implemented to filter the diagnostic result by applying a predetermined condition. Here, the filtering of the diagnostic result by applying the predetermined condition may refer to obtaining a filtered diagnostic result by applying the corresponding condition to the diagnostic result received from the database 100, or may refer to receiving the diagnostic result to which the corresponding condition is applied from the database 100, but is not limited thereto.

Specifically, the filtering unit 310 may filter the diagnostic result by applying a predetermined first condition for at least one of a period, a season, a region, an age, and a target analyte. For example, the filtering unit 310 may provide a selection menu for selecting a period, and apply a period selected by the user through the selection menu to obtain a filtered diagnostic result corresponding to the period from the diagnostic result. For another example, the filtering unit 310 may provide a selection menu for selecting a type (e.g., a virus species name) and a group (e.g., a respiratory virus or an HPV) of the target analyte, and apply the type or group selected by the user through the selection menu to obtain a filtered diagnostic result corresponding to the type or group from the diagnostic result.

In an embodiment, the filtering unit 310 may filter the diagnostic result by applying the predetermined first condition for a type of an infectious disease (e.g., upper airway, lower airway, etc.) and a type of a diagnosis product used in the diagnostic result together. In another embodiment, the filtering unit 310 may filter the diagnostic result by applying the predetermined first condition or a user input for at least one of the qPCR device, the extraction method, the target signaling mechanism, and the polymerase mastermix. For example, the filtering unit 310 may provide a selection menu for selecting a category related to the corresponding information, and apply the category selected by the user through the selection menu to obtain a filtered diagnostic result from the diagnostic result. As described above, the filtering may be performed in various ways, and is not limited to the above-described embodiments. The filtered diagnostic result may be used in a process of generating information on a predetermined item in each of the single detection case and the concurrent detection case in a subsequent operation.

The item value calculation unit 320 may be implemented to calculate a value of a predetermined item in the single detection case and/or the concurrent detection case from the diagnostic results. Specifically, the item value calculation unit 320 may calculate the value of the predetermined item in the single detection case and the concurrent detection case from the diagnostic results obtained by the obtaining unit 210 or the diagnostic results filtered by the filtering unit 310.

More specifically, the item value calculation unit 320 may calculate at least one of: (a) a first C_{T} distribution range and a second C_{T} distribution range for a specific target analyte (e.g., a specific target virus); and (b) a count of a first examinees and a count of a second examinees for a specific target analyte (e.g., a specific target virus) from the diagnostic result.

Here, the first C_{T} distribution range indicates a C_{T} distribution range obtained from a specific target analyte in a single detection case where only the specific target analyte (e.g., the specific target virus) among a plurality of target analytes (e.g., a plurality of target viruses) is detected. In addition, the second C_{T} distribution range indicates a distribution range of C_{T} values obtained from the specific target analyte in a concurrent detection case where one or more other target analytes (e.g., other target viruses) are detected together with the corresponding specific target analyte (e.g., specific target virus) among the plurality of target analytes (e.g., the plurality of target viruses). For example, when the specific target analyte is AdV, the first C_{T} distribution range indicates a C_{T} distribution range for AdV of examinees corresponding to a single infection case infected with AdV only, and the second C_{T} distribution range indicates a distribution range of C_{T} values for AdV of examinees corresponding to a co-infection case infected with another pathogen (e.g., HRV, PIV2) together with the AdV.

In addition, "specific" in the specific target analyte and the specific target virus may be understood as a term for distinguishing a corresponding object from other objects. For example, unlike in the single detection case where only the specific target virus (e.g., AdV) is detected, the specific target virus (e.g., AdV) may be understood as a term for distinguishing the specific target virus (e.g., AdV) from other target viruses (e.g., HRV, PIV2) in the concurrent detection case where other target viruses (e.g., HRV, PIV2) are also detected. In example embodiments, C_{T} values of several concurrently detected target viruses (e.g., AdV, HRV, and PIV2) may be included in each of the diagnostic results corresponding to the concurrent detection case, and C_{T} values of the corresponding specific target virus (e.g., AdV) among them may be applied to the second C_{T} distribution range.

In addition, C_{T} is a cycle threshold used in the technical field, and may be interpreted as meaning a cycle value when a corresponding signal value corresponds to a predetermined threshold in a nucleic acid amplification result including a signal value (e.g., RFU) in a plurality of cycles obtained from a nucleic acid amplification apparatus, but the calculation method or meaning of C_{T} is not limited thereto. For example, C_{T} may be broadly interpreted to include a signal value or a measured value of a predetermined parameter when the nucleic acid amplification result or detection result for the target analyte satisfies a preset condition, or the like. In addition, C_{T} may be interpreted as a meaning indicated by the terms C_{P} (cross point), TOP (take-off point), and C_{Q} (quantization cycle).

In addition, the count of the first examinees indicates a count of examinees corresponding to the single detection case among the plurality of examinees. In addition, the count of the second examinees indicates a count of examinees corresponding to the concurrent detection case. For example, when the specific target virus is AdV, the count of the first examinees indicates the count of examinees corresponding to the single infection case infected only with AdV among the total count of examinees, and the count of the second examinees indicates the count of examinees corresponding to the concurrent infection case infected with another pathogen (e.g., HRV, PIV2) together with AdV among the total count of examinees.

In addition, the count of examinees may be interpreted to mean a value obtained by counting the count of examinees in each of the single detection case and the concurrent detection case. Alternatively, the count of examinees may also be interpreted to mean a ratio of the count of examinees in each of the single detection case and the concurrent detection case with respect to a specific target analyte to the total count of examinees in a diagnostic result (or an entire diagnostic result) targeting the corresponding specific target analyte, but is not limited thereto. For example, the count of examinees may include a prevalence of a specific target virus over a given period of time, the count of cases in which the specific target virus is tested positive, and the like. As described above, values converted in various ways may be used as the count of examinees in order to easily compare relative numerical values between cases.

In addition, a preset target analyte may be applied to the above specific target analyte, a target analyte selected according to a user input may be applied to thereof, and each of the plurality of predetermined target analytes may be applied to thereof sequentially or randomly. For example, the above-described operation may be performed, for each of a plurality of preset target viruses, in a manner of calculating a value of the predetermined item in the single detection case and the concurrent detection case for each corresponding target virus, and in this case, it may be understood that the specific target virus refers to any one of the plurality of target viruses as a target.

The display control unit 330 may be implemented to control display of the calculated value of the predetermined item on a single screen. Specifically, the display control unit 330 may control display of the calculated values of the predetermined items in the single detection case and the concurrent detection case on a single screen of the computer device 300 or the user terminal 200.

More specifically, the display control unit 330 may control display of at least one of (a) the first C_{T} distribution range and the second C_{T} distribution range for the specific target analyte (e.g., the specific target virus); and (b) the count of the first examinees and the count of the second examinees for the specific target analyte (e.g., the specific target virus); on a single screen of the computer device 300 or the user terminal 200.

Various embodiments of the operation/function of the item value calculation unit 320 and the display control unit 330 will be described in more detail below.

### 1) the C_{T} distribution range in the single detection case and the concurrent detection case

The item value calculation unit 320 according to the first embodiment may calculate the first C_{T} distribution range and the second C_{T} distribution range for a specific target analyte from the diagnostic results. In an embodiment, the item value calculation unit 320 may calculate the first C_{T} distribution range and the second C_{T} distribution range for a specific target virus by using the diagnostic results.

In an embodiment, the item value calculation unit 320 may search for diagnostic results corresponding to each of the single detection case and the concurrent detection case for a specific target analyte based on the examinee data, the target analyte data, and the positive/negative determination data included in each diagnostic result. In addition, the item value calculation unit 320 may calculate the first C_{T} distribution range for the corresponding specific target analyte in the single detection case, based on the C_{T} values included in the positive/negative determination data of the diagnostic results corresponding to the searched single detection cases. In addition, the item value calculation unit 320 may calculate the second C_{T} distribution range for the corresponding specific target analyte in the concurrent detection case, based on the C_{T} values included in the positive/negative determination data of the diagnostic results corresponding to the searched concurrent detection cases.

For example, when the specific target virus is AdV, the item value calculation unit 320 may filter the diagnostic results that are determined to be positive for at least AdV from the plurality of diagnostic results, based on the target analyte data and the positive/negative determination data of each of the plurality of diagnostic results. In addition, the item value calculation unit 320 may detect: (a) diagnostic results of a "single detection case for AdV" where only AdV, which is the specific target virus, is determined to be positive from the filtered diagnostic results; and (b) diagnostic results of a "concurrent detection case for AdV" where AdV and one or more other target viruses are determined to be positive together from the filtered diagnostic results;, based on the count of target analyte identifiers included in the target analyte data and the positive/negative determination data for each target analyte, in each of the filtered diagnostic results. In addition, the item value calculation unit 320 may calculate a "first C_{T} distribution range for AdV" including a statistical value that samples C_{T} values obtained from a specific target virus, AdV, which is determined to be positive, based on the positive/negative determination data of each of the diagnostic results of the "single detection case for AdV". Similarly, the item value calculation unit 320 may calculate a "second C_{T} distribution range for AdV" including a statistical value that samples C_{T} values obtained from the specific target virus, AdV, among target viruses that are determined to be positive, based on the positive/negative determination data of each of the diagnostic results of the "concurrent detection case for AdV".

In an embodiment, the statistical value may include at least one of a median value, a first quartile (Q₁), a third quartile (Q₃), an average value, a mode value, an interquartile range (IQR), a minimum value, and a maximum value for the corresponding sample.

Here, the first quartile, the median value, and the third quartile respectively indicate values corresponding to 25%, 50%, and 75% when the sizes of the corresponding samples are arranged in ascending order. In addition, the IQR is a range excluding a 0% to 25% portion and a 75% to 100% portion, which are 1/4 of both ends when the sizes of the corresponding samples are arranged in ascending order, and may represent a difference between the first quartile and the third quartile. In addition, the minimum value may be calculated based on the first quartile and the IQR, for example, may be calculated according to (Q₁ - 1.5 * IQR). In addition, the maximum value may be calculated based on the third quartile and IQR, for example, may be calculated according to (Q₃ + 1.5 * IQR). However, the statistical value is not limited thereto, and may further include various values used for statistical analysis in addition to the above-described embodiments.

Referring to the above-described embodiments, for each of a plurality of target analytes, the item value calculation unit 320 may calculate the first C_{T} distribution range and the second C_{T} distribution range for the corresponding target analyte. For example, the item value calculation unit 320 may calculate: (a) the first C_{T} distribution range and the second C_{T} distribution range for AdV; (b) the first C_{T} distribution range and the second C_{T} distribution range for HRV; and (c) the first C_{T} distribution range and the second C_{T} distribution range for PIV2, and the like, in the above-described manner.

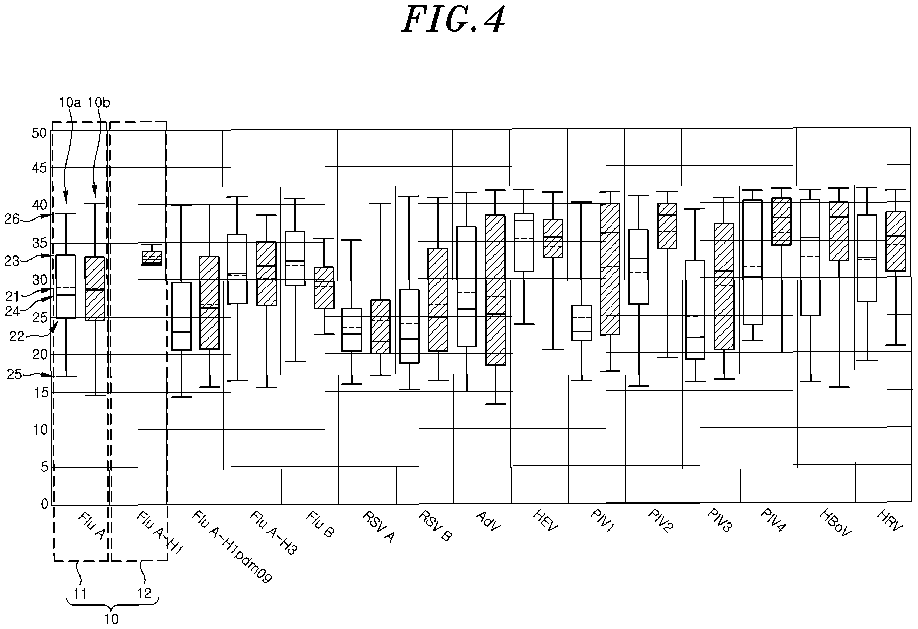

The display control unit 330 according to the first embodiment may control that the calculated first C_{T} distribution range and the calculated second C_{T} distribution range for the specific target analyte are displayed on a single screen of the computer device 300 or the user terminal 200. This will be described with reference to FIG. 4.

FIG. 4 is a diagram exemplarily illustrating that the first C_{T} distribution range and the second C_{T} distribution range for a specific target virus are displayed on a single screen according to the first embodiment.

Referring to FIG. 4, a region 10 corresponding to a specific target virus may be provided on a first screen, and the first C_{T} distribution range in the single detection case and the second C_{T} distribution range in the concurrent detection case for the specific target virus may be displayed together in the region 10. For example, when the specific target virus is Influenza A virus (Flu A), the first C_{T} distribution range in the single detection case for Flu A and the second C_{T} distribution range in the concurrent detection case for Flu A may be displayed together at adjacent positions within the region 10 corresponding to Flu A. In addition, each C_{T} distribution range may be scaled and displayed according to the size of the C_{T} value, based on an axis (e.g., the Y axis) indicating the size of the C_{T} value.

In an embodiment, on the first screen, the region 10 corresponding to each of the plurality of target viruses may be provided. For example, when the count of the above-described plurality is N (N is a natural number), the region 10 corresponding to each of the N target viruses may be a first region 11 corresponding to a first target virus (e.g., Flu A), a second region 12 corresponding to a second target virus (e.g., Flu A-H1), ... , and a Nth region corresponding to an Nth target virus.

In an embodiment, in each region 10, the first C_{T} distribution range in the single detection case and the second C_{T} distribution range in the concurrent detection case for the specific target virus may be displayed in a comparable method. Here, being displayed in the comparable manner may be understood as a concept encompassing various methods in which the predetermined values or ranges between the first C_{T} distribution range and the second C_{T} distribution range for the corresponding specific target virus are processed and displayed in a form that is visually mutually comparable to each other.

In an embodiment, in each region 10, the first C_{T} distribution range and the second C_{T} distribution range may be displayed adjacent to each other within a preset distance. For example, a first graph 10a indicating the first C_{T} distribution range in the single detection case for Flu A and a second graph 10b indicating the second C_{T} distribution range in the concurrent detection case for Flu A may be displayed together at adjacent positions within the first region 11 corresponding to Flu A. In addition, the first C_{T} distribution range and the second C_{T} distribution range may be arranged side by side in the left-right direction or in the top-bottom direction, based on an axis (e.g., the Y axis) indicating the size of the C_{T} value. Accordingly, from the user's point of view, the statistical value of the C_{T} values in the single detection case for the corresponding target virus and the statistical value of the C_{T} values in the concurrent detection case can be easily compared with each other, thereby improving user convenience.

In an embodiment, each region 10 may be aligned based on a first axis (e.g., the X axis) indicating information (e.g., the name of the target virus) on what the specific target virus is and a second axis (e.g., the Y axis) indicating the magnitude of the C_{T} value. For example, the region 10 corresponding to each of the target viruses, such as Flu A, Flu A-H1, Flu A-H1 pdm09, and the like, may be aligned based on the X axis, the name of each corresponding target virus may be displayed along the X axis at the lower end of each region 10, and the first C_{T} distribution range and the second C_{T} distribution range for the corresponding target virus in each region 10 may be scaled and displayed according to the size of the C_{T} value based on the Y axis.

In an embodiment, in each of the first C_{T} distribution range and the second C_{T} distribution range for the specific target virus, at least one of the median value (see identification number 21), the first quartile (see identification number 22), the third quartile (see identification number 23), the average value (see identification number 24), and the mode value may be indicated, and at least one of the IQR, the minimum value (see identification number 25), and the maximum value (see identification number 26) may be further indicated. For example, as shown in FIG. 4, a graph corresponding to a point at which the median value, the first quartile, the third quartile, the average value, and the like, are located in the corresponding sample or a size thereof may be displayed, or the median value, the average value, the standard deviation, the variance, and the like may be displayed as text.

In an embodiment, each of the first C_{T} distribution range and the second C_{T} distribution range for the specific target virus may be displayed in a box plot method. For example, as shown in FIG. 4, IQR may be expressed as a box, the first quartile and the third quartile may be indicated by a boundary line at the end of the box, the median value and the average value may be indicated as a straight line or a dotted line within the box, and the minimum value and the maximum value may be indicated as a dotted line, and the statistical values indicated in this way may be scaled according to the size of the C_{T} value displayed on the Y axis.

Accordingly, there is an advantage in that the user can intuitively compare the median value and the average value of the single detection case and the concurrent detection case for each of the plurality of target viruses. In addition, there is an advantage in that the user can intuitively compare the density of the distribution of C_{T} values corresponding to 25% to 75% in the order of size in the sample through the IQR length expressed as the box. In addition, there is an advantage in that the user can intuitively compare a difference between criteria of outliers that are out of a range of the minimum value and the maximum value.

For example, in the case of the Flu A, the difference between the median value, the average value, and the IQR in each of the case of the single detection case and the concurrent detection case may be relatively intuitively checked by the user, and in the case of the PIV 1 (Parainfluenza virus type 1), the difference may be intuitively checked by the user to be relatively large.

In an embodiment, an identifiable mark may be added for one or more target viruses of which a difference between a representative value of the first C_{T} distribution range and a representative value of the second C_{T} distribution range is not less than a predetermined reference value among the plurality of target viruses. In an embodiment, the identifiable mark may be added in the form of text, a table, a graph, and/or an image, or may be provided in a manner that makes an indication method, such as color, different from other target viruses. For example, in order to distinguish a color or shape of a graph representing the C_{T} distribution range within the region 10 corresponding to the target virus whose difference is greater than a reference value from other graphs, the identifiable mark may be highlighted. Alternatively, the identifiable mark may be provided in a form of a preset icon or the like added to a corresponding screen area. In an embodiment, as the representative value, the above-described median value, average value, mode value, or IQR may be used. For example, (a) the boundary of the region 10 where the average value difference between the first C_{T} distribution range and the second C_{T} distribution range for the corresponding target virus is equal to or greater than the reference value, or (b) the C_{T} distribution range in the corresponding region 10 may be highlighted in a preset color (see reference number 30).

In an embodiment, for at least one target virus of which the count of the second examinees is not less than a predetermined reference value than the count of the first examinees among the plurality of target viruses, when a representative value of the C_{T} distribution range is not less than the representative value of the C_{T} distribution range in the single detection case, an identifiable mark may be added. In an embodiment, the identifiable mark may be a form of a notification message including a sentence expression indicating that both the representative value of the C_{T} distribution range and the count of examinees of the target virus are greater in the concurrent detection case than in the single detection case, or may be a visual indication for directly or indirectly emphasizing the same. The count of the first examinees and the count of the second examinees will be described in detail in the following section of "2) the count of examinees in the single detection case and the concurrent detection case". For example, a notification message may be displayed on the first screen informing that (a) for a specific target virus, a C_{T} value tends to relatively increase as the count of concurrent infection increases compared to the single infection, and (b) there is a tendency that the viral load for the amount of the corresponding target virus in the human body tends to relatively decrease in the concurrent infection compared to the single infection.

In an embodiment, when the target virus is a parainfluenza virus (PIV)-based virus and the count of the second examinees for the target virus is not less than the count of the first examinees for the target virus, an additional identifiable mark may be provided. For example, as shown in FIG. 4, in the case of the PIV-based virus, a notification message indicating that the C_{T} value difference between the single detection case and the concurrent detection case tends to relatively increase more than others or a visual indication for directly or indirectly emphasizing the notification message may be added to the first screen. As described above, the user may be provided with various insight based on a difference in the C_{T} distribution range between the single detection case and the concurrent detection case through the notification message.

In an embodiment, each region 10 may be displayed sorted in ascending or descending order based on the difference between the representative value of the first C_{T} distribution range and the representative value of the second C_{T} distribution range for the corresponding specific target virus. For example, the regions, from the region 10 for the PIV1 having the largest difference in average value to the region 10 for the Flu A having the smallest difference, may be aligned and displayed from left to right (or from top to bottom).

In an embodiment, when a selection input for the regions 10 is received, a window displaying the first C_{T} distribution range and the second C_{T} distribution range in the corresponding region 10 expanded at a preset ratio may be provided. For example, the first graph 10a indicating the first C_{T} distribution range and the second graph 10b indicating the second C_{T} distribution range are displayed in each region 10, and when a selection input for the first graph 10a or the second graph 10b is received, each statistical value may be displayed on the corresponding graph in an overlapping manner. In addition, information on the sample used for calculating each C_{T} distribution range (e.g., the count of all examinees corresponding to the sample, a period applied to the sample, etc.) may be displayed together on the corresponding window or graph.

In one embodiment, the first C_{T} distribution range and the second C_{T} distribution range for the corresponding specific target virus in each region 10 may be displayed in different ways. For example, colors, patterns, or the like of the first graph 10a and the second graph 10b may be different, and the first graph 10a and the second graph 10b may each display a predetermined symbol or text indicating the single detection case and the concurrent detection case, respectively.

In an embodiment, the C_{T} distribution range displayed in each region 10 may be displayed in a different manner for each region 10. For example, a different color may be matched to each target virus corresponding to each region 10, and the box plot graph or representative value of the first C_{T} distribution range and the second C_{T} distribution range in each region 10 may be displayed in a color matched to the corresponding target virus.

In an embodiment, a color matching the corresponding target virus may be determined based on a difference between the first C_{T} distribution range and the second C_{T} distribution range. For example, the target virus having a large difference between the median values of the first C_{T} distribution range and the second C_{T} distribution range may be matched to a color closer to a first color (e.g., red) among a plurality of preset colors, and the target virus having a small difference may be matched to a color closer to a second color (e.g., blue).

Although various embodiments have been described above with reference to FIG. 4 in which the C_{T} distribution range is indicated according to the box plot method, the present disclosure is not limited thereto, and various statistical analysis methods may be applied. For example, the above-described C_{T} distribution range may be displayed as various types of diagrams according to various methods such as a histogram, a normal distribution, a frequency distribution, and the like.

In addition, values for various items described throughout the specification, including the C_{T} distribution range, may be represented in various forms such as a multidimensional diagram (e.g., an image, a graph, a matrix), and the like, but are not limited thereto. For example, a two-dimensional graph image for the C_{T} distribution range may be generated and displayed, the statistical values of the C_{T} distribution range may be displayed in a way that they are plotted on a graph based on a graphic interface for the graph. In addition, various general-purpose algorithms required for such image processing or graphic processing may be included in the display control unit 330.

### 2) the count of examinees in the single detection case and the concurrent detection case

The item value calculation unit 320 according to the second embodiment may calculate the count of the first examinees and the count of the second examinees for a specific target analyte from the diagnostic results. In an embodiment, the item value calculation unit 320 may calculate the count of the first examinees and the count of the second examinees for the specific target virus by using the diagnostic results.

In an embodiment, the item value calculation unit 320 may search for diagnosis results performed on at least a specific target analyte based on the target analyte data included in each diagnostic result. In addition, the item value calculation unit 320 may calculate the count of the first examinees corresponding to the single detection case for the specific target analyte and the count of the second examinees corresponding to the concurrent detection case for the specific target analyte, respectively, based on the examinee data, the target analyte data, and the positive/negative determination data in each of the searched diagnostic results.

For example, when the specific target virus is AdV, the item value calculation unit 320 may filter the diagnostic results targeting at least AdV from the plurality of diagnostic results, based on the target analyte data of each of the plurality of diagnostic results. In addition, the item value calculation unit 320 may calculate the total count of examinees whose diagnostic results are performed for detecting at least AdV as a target, based on the examinee identifier in each of the filtered diagnostic results. In addition, the item value calculation unit 320 may detect (a) diagnostic results of a "single detection case for AdV" in which only AdV, which is the specific target virus, is determined to be positive from the filtered diagnostic results and (b) diagnostic results of a "concurrent detection case for AdV" in which AdV and one or more other target viruses are determined to be positive together from the filtered diagnostic results, based on the examinee identifier, the count of target analyte identifiers, and the positive/negative determination data for each target analyte in each of the filtered diagnostic results. Also, the item value calculation unit 320 may calculate the count of first examinees for AdV, by counting the count of diagnostic results of the "single detection case for AdV" or by counting the count of examinees who are determined to be positive for only AdV, which is the specific target virus, based on each examinee identifier. Likewise, the item value calculation unit 320 may calculate the count of the second examinees for AdV, by counting the count of diagnostic results of the "concurrent detection case for AdV" or by counting the count of examinees who are determined to be positive for AdV and another target virus together based on each examinee identifier.

Referring to the above-described embodiments, for each of the plurality of target analytes, the item value calculation unit 320 may calculate the count of the first examinees and the count of the second examinees for the corresponding target analyte. For example, the item value calculation unit 320 may calculate: (a) the count of the first examinees and the count of the second examinees for AdV; (b) the count of the first examinees and the count of the second examinees for HRV; and (c) the count of the first examinees and the count of the second examinees for PIV2;, and the like, in the above-described manner.

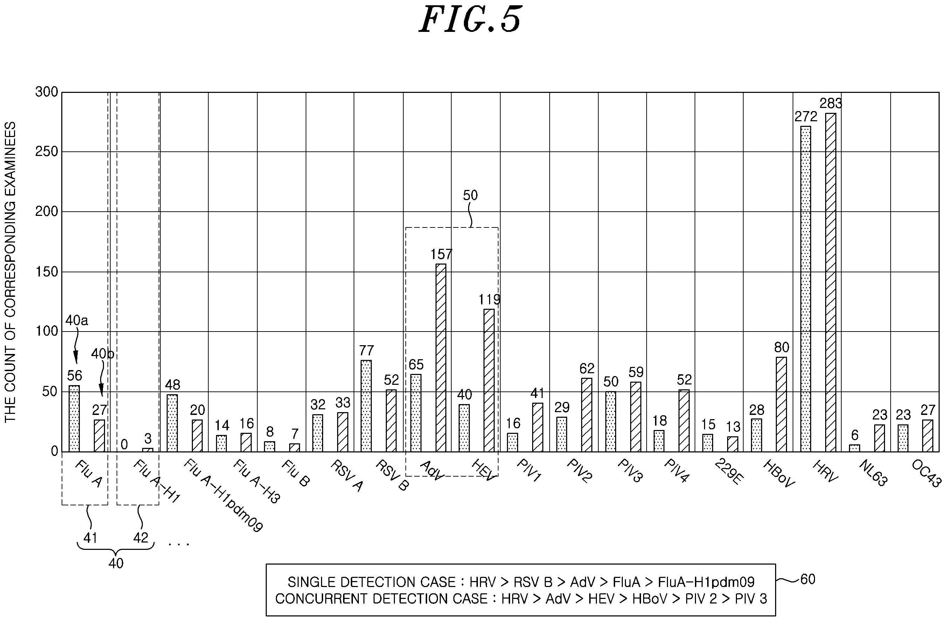

The display control unit 330 according to the second embodiment may control that the calculated count of the first examinees and the calculated count of the second examinees for the specific target analyte are displayed on a single screen of the computer device 300 or the user terminal 200. This will be described with reference to FIG. 5.

FIG. 5 is a diagram exemplarily illustrating that the count of the first examinees and the count of the second examinees for the specific target virus are displayed on a single screen according to the second embodiment.

Referring to FIG. 5, the region 40 corresponding to a specific target virus may be provided on a second screen, and the count of the first examinees in the single detection case for the specific target virus and the count of the second examinees in the concurrent detection case for the specific target virus may be displayed together on the region 40. For example, when the specific target virus is Flu A, the count (e.g., 56) of the first examinees in the single detection case for Flu A and the count (e.g., 26) of the second examinees in a concurrent detection case for Flu A may be displayed together at adjacent positions within the region 40 corresponding to Flu A. In addition, a bar graph indicating each count of the examinee may be scaled and displayed according to the size, based on an axis (e.g., the Y-axis) indicating the size of the count of examinees.

In one embodiment, the region 40 corresponding to each of the plurality of target viruses may be provided on the second screen. Likewise, the region 40 may include a first region 41 corresponding to the first target virus, a second region 42 corresponding to the second target virus, ··· , and a Nth region corresponding to the Nth target virus. In an embodiment, in each region 40, the count of the first examinees in the single detection case for the target virus and the count of the second examinees in the concurrent detection case may be displayed in a comparable manner. In an embodiment, in each region 40, the count of the first examinees and the count of the second examinees may be displayed adjacent to each other within a preset distance.

For example, a third graph 40a indicating the count of the first examinees in the single detection case for Flu A and a fourth graph 40b indicating the count of the second examinees in the concurrent detection case for Flu A may be displayed together at adjacent positions in the first region 41 corresponding to Flu A. In addition, the third graph 40a and the fourth graph 40b may be arranged side by side in the left-right direction or in the top-bottom direction, based on an axis (e.g., the Y-axis) indicating the size of the count of the corresponding examinees. Accordingly, from the user's point of view, the count of patients in the single detection case and the count of patients in the concurrent detection case for the corresponding target virus can be easily compared with each other, thereby improving user convenience.

In an embodiment, each region 40 may be aligned based on a first axis (e.g., an X axis) indicating information (e.g., the name of the target virus) on what the corresponding target virus is and a second axis (e.g., a Y axis) indicating the size of the count of the corresponding examinees.

In an embodiment, in the third graph 40a and the fourth graph 40b for the corresponding target virus in each region 40, the count of the first examinees and the count of the second examinees may be displayed together as text. For example, as shown in FIG. 5, the third graph 40a and the fourth graph 40b in each region 40 may be displayed in a bar graph format in proportion to the count of the corresponding examinees, and the count of the corresponding examinees may be displayed as text in each graph.

In an embodiment, the count of the first examinees and the count of the second examinees for the corresponding target virus in each region 40 may be displayed in a different manner. Likewise, the count of examinees indicated in each region 40 may be displayed in a different manner for each region 40.

In an embodiment, an identifiable mark may be added for one or more target viruses of which a difference between the count of the first examinees and the count of the second examinees is not less than a predetermined reference value among the plurality of target viruses. As described above, the identifiable mark may be provided in the form of text, a graph, and/or an image. For example, (a) the boundary of the region 40 where the difference between the count of the first examinees and the count of the second examinees is equal to or greater than the reference value, or (b) the third graph 40a and the fourth graph 40b in the corresponding region 10 may be highlighted in a preset color (see reference number 50).

In an embodiment, for at least one target virus of which the difference between the first C_{T} distribution range and the second C_{T} distribution range is not less than the reference value among the plurality of target viruses, when the count of corresponding examinees is larger in the concurrent detection case than in the single detection case, an identifiable mark may be added. In an embodiment, the identifiable mark may be a form of a notification message including a sentence expression indicating that both the count of examinees of the target virus and the representative value of the C_{T} distribution range are greater in the concurrent detection case than in the single detection case, or may be a visual indication for directly or indirectly emphasizing the same. For example, a notification message may be displayed on the second screen informing that (a) for a specific target virus, as the average value of C_{T} values in the concurrent infection compared to the single infection increases, the count of examinees in the concurrent infection compared to the single infection tends to relatively increase, and (b) there is a tendency that the viral load for the amount of the corresponding target virus in the human body tends to relatively decrease in the concurrent infection compared to the single infection. In addition, in one embodiment, when the specific target virus is the PIV-based virus, an additional identifiable mark may be provided. Similarly, in the case of the PIV-based virus, a message indicating that there is a tendency that the difference in the count of the corresponding examinees is relatively increased or a visual indication for directly or indirectly emphasizing the same may be additionally provided on the second screen.

In an embodiment, each region 40 may be displayed sorted in ascending or descending order based on the difference between the count of the first examinees and the count of the second examinees for the specific target virus. Further, in an embodiment, the count of the first examinees and the count of the second examinees for the specific target virus in each region 40 may be displayed in a different manner.

In an embodiment, the count of the first examinees and the count of the second examinees may be calculated as a ratio of the count of the corresponding examinees to the total count of examinees, and the third graph 40a and the fourth graph 40b in each region 40 may be scaled and displayed according to each ratio. For example, when (a) the total count of examinees in the diagnostic results targeting Flu A is 3500, (b) the count of examinees who are tested positive for only Flu A is 56, and (c) the count of examinees who are tested positive for another virus together with Flu A is 27, the count of the first examinees may be calculated as 1.6(%) (=56/3500), and the count of the second examinees may be calculated as about 0.8(%) (=27/3500). In this case, in each region 40, the lengths of the third graph 40a and the fourth graph 40b may be scaled and displayed according to a Y axis where the total size is 100(%) or a predetermined ratio value. Accordingly, there is an advantage in that the user can intuitively compare a relative value for each of the count of the first examinees and the count of second examinees among the total count of examinees in the diagnostic results performed for detecting at least the corresponding target virus.

In an embodiment, on the second screen, the regions 40 may be displayed in an arranged manner such that the count of the first examinees indicated in each region (40) is arranged in ascending or descending order. For example, the regions, from the region 40 for the HRV having the largest count of the first examinees to the region 40 for the Flu A-H1 having the smallest count, may be aligned and displayed from left to right (or from top to bottom).

In an embodiment, on the second screen, the names of the corresponding target viruses may be displayed in an arranged manner such that the count of the first examinees is arranged in ascending or descending order. For example, in the case of the single detection case, "HRV > RSVB > AdV > FluA > FluA-H1pdm09 > ··· > FluA-H1", in which the names from the target virus having the largest count of the first examinees to the target virus having the smallest count are sequentially listed, may be output as a message at the lower end of the region 40 (see reference number 60).

In an embodiment, on the second screen, the names of the corresponding target viruses may be displayed in an arranged manner such that the count of the second examinees is arranged in ascending or descending order. For example, in the case of the concurrent detection case, "HRV > AdV > HEV > HboV > PIV2 > PIV3 > ··· > FluA-H1", in which the names from the target virus having the largest count of the second examinees to the target virus having the smallest count are sequentially listed, may be output as a message at the lower end of the region 40 (see reference number 60).

In an embodiment, when a selection input for the regions 40 is received, a window in which the statistical value in the single detection case and the statistical value in the concurrent detection case for the corresponding target virus are displayed together may be provided. For example, when a selection input for the third graph 40a or the fourth graph 40b displayed in each region 40 is received, (a) a first statistical value in the single detection case and (b) a second statistical value in the concurrent detection case, which sample statistical values from the diagnostic results targeting a specific target virus corresponding to the selected region 40, may be calculated. In addition, a window in which the first statistical value and the second statistical value are displayed in a comparable manner may be displayed overlapping the corresponding region 40. In an embodiment, when a selection input for the regions 40 is received, a window in which the first C_{T} distribution range and the second C_{T} distribution range for the corresponding target virus are displayed may be provided.

According to an embodiment, the second screen may be provided in another region within the same screen where the first screen is displayed, or may be provided in a separate screen.

Although various embodiments have been described above with reference to FIG. 5 in which the count of examinees is displayed according to the bar graph method, the present disclosure is not limited thereto, and various display methods may be applied. For example, the count of examinees described above may be displayed as various types of diagrams such as a circle graph, a band graph, a graph of broken lines, etc.

### 3) Information about one or more other target analytes detected together with the specific target analyte in the concurrent detection case

The item value calculation unit 320 according to the third embodiment may obtain information about one or more other target analytes detected together with a specific target analyte in the concurrent detection case by using the diagnostic results. In an embodiment, the item value calculation unit 320 may obtain information about one or more other target viruses detected together with a specific target virus in the concurrent detection case by using the diagnostic results.

In the third embodiment, the information about one or more other target viruses detected together with the specific target virus in the concurrent detection case may include: (a) a name of each of the one or more other target viruses detected together with the specific target virus; and (b) a count of examinees and/or a C_{T} distribution range in the concurrent detection case in which each of the one or more other target viruses are detected together with the specific target virus. For example, when the specific target virus is HRV, the above information may include: (a) names of AdV, HEV, PIV3, and the like as other target viruses detectable together with HRV; (b) the count of examinees in each concurrent detection case where each of AdV, HEV, and PIV3 was detected together with HRV; and (c) the C_{T} distribution range for HRV.

Specifically, the item value calculation unit 320 may search for diagnostic results in which the specific target virus (e.g., HRV) and one or more other target viruses are determined to be positive together, based on the examinee data, the target analyte data, and the positive/negative determination data included in each diagnostic result. In addition, the item value calculation unit 320 may classify the searched diagnostic results by each corresponding other target virus, based on the target analyte identifiers of other target viruses (e.g., AdV, HEV, and PIV3) that may be determined to be positive together with the specific target virus. In addition, the item value calculation unit 320 may count the count of diagnostic results classified for each corresponding other target virus (e.g., AdV, HEV, or PIV3) to calculate (a) the count of examinees whose each other target virus is detected together with the specific target virus (e.g., HRV), or (b) the C_{T} distribution range that samples C_{T} values of the specific target virus (e.g., HRV) from the diagnostic results classified for each other target virus. The examples of calculating the above count of examinees or the above C_{T} distribution range may be understood with reference to the above-described embodiments, and reduplicated descriptions thereof will be omitted.

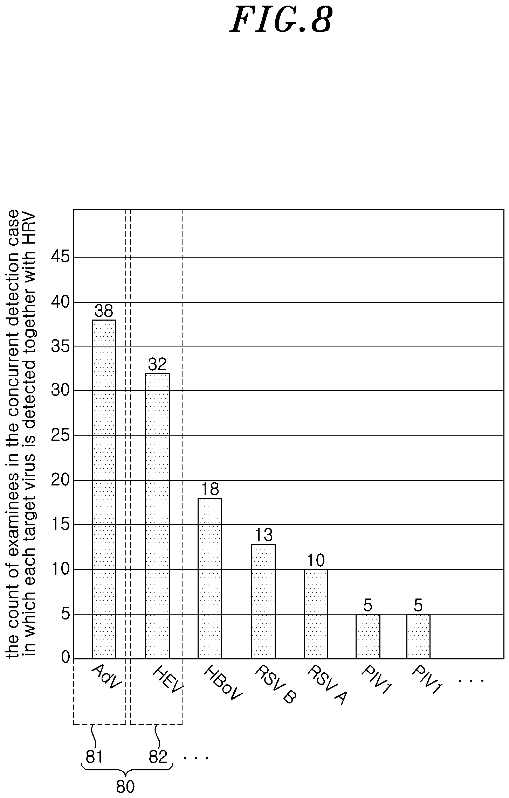

In an embodiment 3-1, the display control unit 330 may control display of the information about one or more other target viruses detected together with the specific target virus in the concurrent detection case on a single screen of the computer device 300 or the user terminal 200. This will be described with reference to FIG. 8

FIG. 8 is a diagram exemplarily illustrating that information about one or more other target viruses detected together with the specific target virus in the concurrent detection case are displayed on a single screen according to the third embodiment.

Referring to FIG. 8, a region 80 corresponding to each of one or more other target viruses detected together with the specific target virus may be provided on a third screen, and the count of examinees in the concurrent detection case for the corresponding target virus may be displayed in the corresponding region 80. For example, when the specific target virus is HRV, the corresponding region 80 may include a first region 81 corresponding to AdV detected together with HRV, a second region 82 corresponding to HEV detected together with HRV, and the like. In addition, (a) the name (e.g., AdV) of the corresponding other target virus and (b) the count (e.g., 38) of examinees in the concurrent detection case in which the corresponding other target virus is tested positive together with HRV may be displayed in each region 80. The above count of examinees may include the count of examinees in the concurrent detection case in which one or more additional target viruses are tested positive together in addition to HRV and the corresponding other target virus (e.g., AdV).

In an embodiment 3-2, the above information about the one or more other target viruses detected together with the specific target virus in the concurrent detection case may include: (a) names of a plurality of combinations for the one or more other target viruses detected together with the specific target virus; and (b) a count of examinees and/or the C_{T} distribution range in the concurrent detection case in which each of the plurality of combinations are detected together with the specific target virus. For example, when the specific target virus is HRV, the above information may include: (i) the names of combinations of other detectable target viruses together with HRV, such as AdV-HEV, HEV-PIV3, and AdV-PIV3 (or HRV-AdV-HEV, HRV-HEV-PIV3, and HRV-AdV-PIV3); (ii) the count of examinees in the concurrent detection case in which each of the above combinations is detected; and (iii) the C_{T} distribution range for HRV.

Specifically, the item value calculation unit 320 may search for diagnostic results in which the specific target virus (e.g., HRV) and one or more other target viruses are determined to be positive together, based on the examinee data, the target analyte data, and the positive/negative determination data included in each diagnostic result. In addition, the item value calculation unit 320 may obtain the plurality of combinations by combining all other target viruses (e.g., AdV, HEV, and PIV3) that may be determined to be positive with the specific target virus, based on the type data of the target virus stored in advance. In addition, the item value calculation unit 320 may classify the searched diagnostic results by each combination by using the target analyte identifiers of the target viruses belonging to each combination. In addition, the item value calculation unit 320 may calculate the count of examinees corresponding to each combination by counting the count of classified diagnostic results, or calculate the C_{T} distribution range that samples C_{T} values of the specific target virus (e.g., HRV) from the diagnostic results classified according to each combination.

In the embodiment 3-2, the display control unit 330 may control display of the above information about one or more other target viruses detected together with the specific target virus in the concurrent detection case on a fourth screen. In a similar manner to that shown in FIG. 8, region 80 corresponding to each of the plurality of combinations may be provided on the fourth screen, and the name of the corresponding combination and the count of examinees in the concurrent detection case for the corresponding combination may be displayed in each region 80. For example, when the specific target virus is HRV, the corresponding region 80 may include a first region 81 corresponding to a first combination of HRV-AdV, a second region 82 corresponding to a second combination of HRV-HEV, a third region corresponding to a third combination of HRV-AdV-HEV, and the like. In addition, (a) the name of the corresponding combination and (b) the count of examinees in the concurrent detection case in which the target viruses of the corresponding combination are determined to be positive together with the HRV may be displayed in each region 80. The above count of examinees refers to the count of examinees in the concurrent detection case in which only the target viruses belonging to the corresponding combination are determined to be positive together with the HRV.

In an embodiment, the process for controlling the display of the information about one or more other target viruses detected together with the specific target virus in the concurrent detection case may be performed corresponding to selection of a single screen displaying the second C_{T} distribution range or the count of the second examinees. Specifically, after the first and second C_{T} distribution ranges or the count of the first and the second examinees are displayed on a one screen, when a user input for selecting the screen region displaying the second C_{T} distribution range or the count of the second examinees is received, the information about one or more other target viruses detected together with the specific target virus in the concurrent detection case may be displayed on the above one screen or another one screen as a response to the corresponding user input.

As a specific example, the third screen or the fourth screen may be provided in connection with the first screen or the second screen. For example, when (a) the first and second C_{T} distribution range (or the count of the first and the second examinees) for the specific target virus are displayed in the form of a bar graph on the first screen (or the second screen), and (b) a user input for selecting the bar graph indicating the second C_{T} distribution range (or the count of the second examinees) in the first screen (or the second screen) is received, as a response to the corresponding user input, (i) the names of each of one or more other target viruses detected together with the specific target virus in the concurrent detection case and (ii) the C_{T} distribution range (or count of examinees) for the specific target virus in the concurrent detection case in which each of the other target viruses was detected together with the specific target virus may be displayed on the third screen. For example, the third screen may be displayed overlapping the first screen or the second screen, may be displayed at a position adjacent to the first screen or the second screen, or may be displayed as a separate screen.

In an embodiment, the selection of the screen region displaying the second C_{T} distribution range or the count of the second examinees may include reception of the user input for selecting the screen region through an input device such as a mouse or a keyboard. In addition, the screen region displaying the second C_{T} distribution range or the count of the second examinees may correspond to a graph region indicating the second C_{T} distribution range or the count of the second examinees, a text region in which the name of the specific target virus or the name of the combination is displayed, or a menu region for displaying the third screen or the fourth screen.

In another embodiment, the third screen or the fourth screen may be provided based on a user input (e.g., a selection menu) for requesting to provide the third screen or the fourth screen regardless of whether the first screen or the second screen is provided, or may be provided together when the first screen or the second screen is provided.

### 4) the count of examinees for each combination of target analytes in the concurrent detection case

The item value calculation unit 320 according to the fourth embodiment may obtain information about the concurrent detection case of the specific target virus and information about the concurrent detection case of an additional specific target virus by using the diagnostic results. Specifically, the item value calculation unit 320 may obtain information about one or more other target viruses detected together with a first specific target virus, information about one or more other target viruses detected together with a second specific target virus, ···, and information about one or more other target viruses detected together with a Nth specific target virus. In addition, the corresponding information may be displayed together on a single screen.

The information about the one or more other target viruses according to an embodiment may include (a) names of combinations of two or more target viruses among the plurality of target viruses, (b) the count of examinees corresponding to the concurrent detection case for each combination, and/or (c) the C_{T} distribution range of the corresponding specific target virus.

In an embodiment, the item value calculation unit 320 may search for diagnostic results determined to be positive for two or more target viruses, based on the examinee data, the target analyte data, and the positive/negative determination data included in each diagnostic result. In addition, the item value calculation unit 320 may calculate the count of examinees corresponding to the concurrent detection cases for each combination, based on combinations of two or more different target viruses as targets, based on the target analyte identifier of each detected diagnostic result.

For example, the item value calculation unit 320 may filter the diagnostic results tested positive for two or more target analytes based on the count of target analyte identifiers and the positive/negative determination data for each target analyte, and derive the plurality of combinations including a first combination of AdV and HRV, a second combination of HEV and HRV, and a third combination of HRV and PIV3, from the filtered diagnostic results. In this case, the count of target analytes to be combined may be two, three, or more. In addition, the item value calculation unit 320 may calculate the count of examinees corresponding to each of the plurality of combinations, by counting the count of diagnostic results corresponding to each of the plurality of combinations from the filtered diagnostic results, or by counting the count of examinees who are determined to be positive for the corresponding combination based on each examinee identifier.

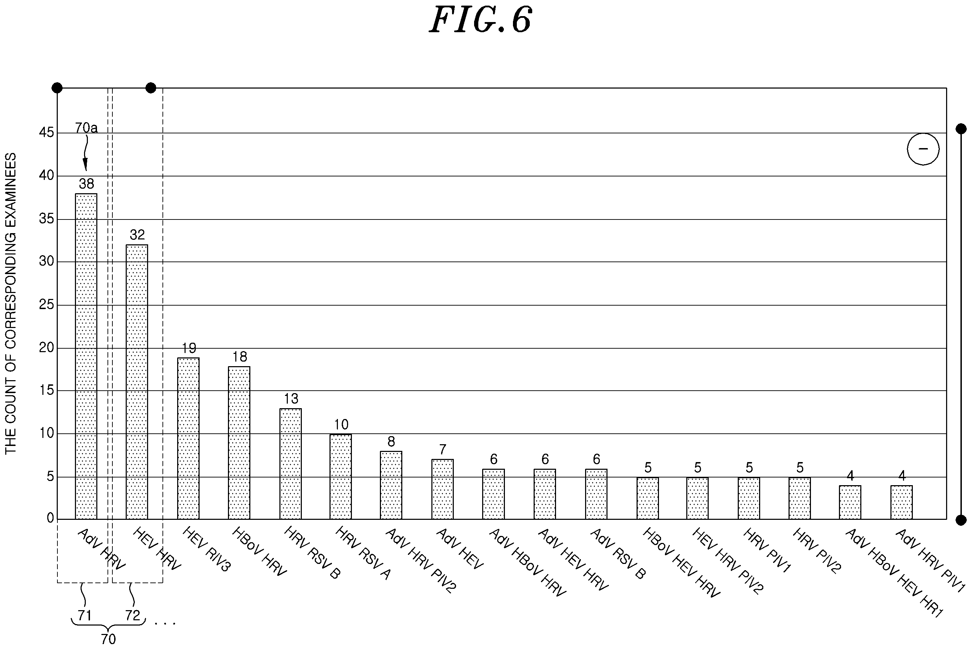

The display control unit 330 according to the fourth embodiment may control that the calculated count of examinees corresponding to each of the plurality of combinations is displayed on a single screen of the computer device 300 or the user terminal 200. This will be described with reference to FIG. 6.

FIG. 6 is a diagram exemplarily illustrating that information about the concurrent detection case of the specific target virus and information about the concurrent detection case of an additional specific target virus are displayed on a single screen according to the fourth embodiment.

Referring to FIG. 6, the region 70 corresponding to each combination of two or more target viruses among the plurality of target viruses may be provided on a fifth screen. For example, the region 70 may be a first region 71 corresponding to the first combination, a second region 72 corresponding to the second combination, ··· , an Nth region corresponding to the Nth combination.

In an embodiment, the count of examinees corresponding to the concurrent detection case for the combination of two or more target viruses may be displayed in each region 70. For example, 71 for the first combination of AdV and HRV, the count (e.g., 38) of examinees who are tested positive for AdV and HRV may be displayed in the first region. In addition, for the second combination of HEV and HRV, the count (e.g., 32) of examinees who are tested positive for HEV and HRV may be displayed in the second region 72. Similarly, a fifth graph 70a (e.g., a bar graph) indicating the count of examinees for each combination may be scaled and displayed according to the size, based on an axis (e.g., the Y axis) indicating the size of the count of examinees.

In an embodiment, the position of each region 70 in the fifth screen may be arranged such that the count of the examinees in the concurrent detection case for the corresponding target virus is arranged in ascending or descending order. For example, the combinations, from AdV-HRV which is a combination of the target viruses in the concurrent detection case having the largest count of the examinees among the concurrent detection cases to AdV-HRV-PIV1 which is a combination of the target viruses having the smallest count of the examinees, may be aligned and displayed from left to right (or from top to bottom).

In an embodiment, the count of examinees indicated in each region 70 may be displayed in a different manner for each region 10. Likewise, a different color may be matched to each of the combinations of the target viruses, and the fifth graph 70a indicating the count of corresponding examinees may be displayed according to the matched colors.

In an embodiment, when a selection input for the region 70 is received, a window displaying the statistical value of the concurrent detection case for the combination of the corresponding target virus may be provided. For example, when the fifth graph 70a indicating the count of examinees for the combination of AdV-HRV is selected by the user, (a) a statistical value in the single detection case for each of AdV and HRV, (b) a statistical value for AdV in the concurrent detection case where AdV and another target virus are detected together, and (c) a statistical value for HRV in the concurrent detection case where HRV and another target virus are detected together may be calculated. In addition, the window in which the calculated statistical values are displayed in a comparable manner may be displayed overlapping the corresponding region 70.

Likewise, various display methods may be applied to the process in which the value or ratio corresponding to the count of third examinees is displayed, and various embodiments described throughout the specification may be applied in a similar manner.

In an embodiment, when any one specific target viruses is selected, display of information about the concurrent detection case of the selected any one specific target virus may be controlled. Specifically, information about the concurrent detection case of each of the first specific target virus to the Nth specific target virus may be displayed on the fifth screen, a selection menu for selecting any one of the first specific target virus to the Nth specific target virus may be provided through the fifth screen. When a user input for selecting any one specific target virus is received through the selection menu, information about the concurrent detection case of the selected specific target virus may be displayed on the fifth screen or a separate screen. For example, as shown in FIG. 6, in each region 70 of the fifth screen, the name of the target virus corresponding to each region 70 may be displayed. When a user input for selecting any one of the names is received, (a) the names of other target viruses detected together with the specific target virus corresponding to the selected name and (b) the count of examinees in the corresponding concurrent detection case may be provided as shown in FIG. 8.

### 5) the count of examinees for each count of concurrently detected target analytes and a count of combinations for the same

The item value calculation unit 320 according to the fifth embodiment may calculate the count of concurrently detected target analytes, the count of corresponding examinees, and the count of combinations of corresponding target analytes, from the diagnostic result. For example, the item value calculation unit 320 may detect diagnostic results tested positive for each of two, three, ···, and M (M is a natural number) target viruses, respectively, based on the count of target analyte identifiers and the positive/negative determination data for each target analyte, and calculate the count of examinees corresponding to each of the detected diagnostic results. In addition, the item value calculation unit 320 may detect a combination of concurrently detected target viruses for each count of concurrently detected target viruses, based on the target analyte identifier of each detected diagnostic result. For example, when the count of concurrently detected target viruses is 2, a combination of target viruses such as AdV-HRV, HEV-HRV, HRV-PIV3, ···, and the like, may be detected. Likewise, the count of the corresponding examinees and the count of the corresponding combinations of the target viruses may be calculated as a value or a ratio of the count to the total count.

The display control unit 330 according to the fifth embodiment may control that the count of concurrently detected target analytes, the count of corresponding examinees, and the count of combinations of corresponding target analytes are displayed on a single screen of the computer device 300 or the user terminal 200.

Various embodiments in which the value for the predetermined item in the single detection case and/or the concurrent detection case is calculated by the item value calculation unit 320 and the screen display of the calculated value is controlled by the display control unit 330 have been described above. However, the predetermined item is not limited to the above-described embodiment. In addition, the operation order of the item value calculation 320 and the display control unit 330 may be sequentially performed for each item, or may be performed according to a predetermined order for several items, but is not limited thereto.

The recommendation information providing unit 340 may be implemented to provide meaningful information to the user by using the calculated value of the predetermined item in each of the single detection case and/or the concurrent detection case.

In an embodiment, the recommendation information providing unit 340 may combine candidate target viruses that may participate in a simultaneous amplification reaction to configure a multiplex NAAT panel, by using the count of examinees and/or the C_{T} distribution range. In an embodiment, such the candidate target virus may be used to construct the multiplex NAAT panel or a molecular diagnostic product for simultaneously detecting the presence or absence of two or more of a plurality of target viruses. Specifically, the result of combining the candidate target viruses may be used as recommendation information for the configuration of two or more target viruses to be simultaneously detected in the multiplex NAAT panel or the molecular diagnostic product. For example, as the candidate target viruses for constituting the multiplex NAAT panel, a result of combining AdV, HEV (hepatitis E virus), PIV1, PIV2, PIV4, HBoV (human bocavirus), and NL63 in which the count of the second examinees is equal to or greater than a predetermined level compared to the count of the first examinees may be provided as the recommendation information.

In an embodiment, the recommendation information providing unit 340 may search for one or more target viruses for which the difference between the count of the first examinees and the count of the second examinees satisfies a predetermined second condition, from among the plurality of target viruses. In an embodiment, the second condition may include at least one of: (a) a condition in which a ratio of the count of the second examinees to the count of the first examinees is not less than a predetermined value; (b) a condition in which the count of the second examinees is not less than a predetermined value; and (c) a condition in which a value obtained by subtracting the count of the first examinees from the count of the second examinees is not less than a predetermined value. For example, as illustrated in FIG. 5, AdV, HEV, HBoV, and the like may be searched as target viruses in which the ratio of the count of the second examinees to the count of the first examinees (e.g., the count of the second examinees / the count of the first examinees) is equal to or greater than a predetermined value (e.g., 2).

In an embodiment, when the count of the searched target viruses is two or more, the recommendation information providing unit 340 may combine the candidate target viruses by using the names of the two or more searched target viruses and the difference between the count of the first examinees and the count of the second examinees. For example, the recommendation information providing unit 340 may calculate a score for each candidate target virus by using the difference between the count of the first examinees and the count of the second examinees, and may calculate the score to be higher (a) as the count of the second examinees is larger than the count of the first examinees and (b) as the difference between the representative values of the first and second C_{T} distribution ranges is larger. In addition, the recommendation information providing unit 340 may generate a plurality of combinations by combining the candidate target viruses, and calculate (e.g., sum up, average) scores of the candidate target viruses belonging to each combination in a predetermined manner to calculate a recommendation score for each combination. According to an embodiment, the combination result may include the plurality of combinations of AdV, HEV, and HBoV and the recommendation score for each of them, and may further include a message recommending that the multiplex NAAT panel be configured by using a combination having the recommendation score higher than a predetermined level.

In an embodiment, the recommendation information providing unit 340 may combine the candidate target viruses by using at least one of a period, a season, a region, and an age in which each of the plurality of target viruses is detected. For example, for each target virus, a virus pandemic score indicating whether a pandemic occurs that the count of examinees exceeds a reference value in at least one of a predetermined period, season, region, and age based on a target time (e.g., a current time point) may be calculated, and the virus pandemic score may be assigned as a weight in the process of combining candidate target viruses. For example, in the case of a target virus that is currently in epidemic, as the virus pandemic score is calculated to be high, even if the count of the second infected persons is relatively small, a relatively large weight may be applied to calculate a high score.

In an embodiment, the recommendation information providing unit 340 may combine the candidate target viruses by using a concurrent detection frequency that each of the plurality of target viruses has for each of the other target viruses. Here, the concurrent detection frequency means the count of examinees corresponding to a case in which the corresponding target virus is detected together with any other target virus in the concurrent detection case. For example, by counting the count of examinees in the concurrent detection cases where AdV was detected with HRV, HEV and PIV3, respectively, the concurrent detection frequencies that AdV has for HRV, HEV and RSV B, respectively, may be calculated as 38, 32 and 6, respectively (see FIG. 6). This concurrent detection frequencies may be assigned as a weight when combining the candidate target viruses, for example, when AdV and HRV are included in the combination, a higher weight may be applied than when AdV and RSV B are included in the combination, and thus a higher recommendation score may be calculated.

In an embodiment, when the C_{T} distribution range or the count of examinees described above is displayed, the recommendation information providing unit 340 may control that the score and/or the combination result are/is displayed together on the corresponding screen. Alternatively, the recommendation information providing unit 340 may generate the combination result as a response to a user input requesting provision of the recommendation information and control that the combination result is displayed on the fourth screen.

In an embodiment, the recommendation information providing unit 340 may provide a viral load to each of the single detection case and the concurrent detection case by using the above-described C_{T} distribution range. Here, the viral load may indicate the degree to which a specific target virus affects the human body.

In an embodiment, a first viral load in the single detection case for a specific target virus may be calculated based on the representative value of the first C_{T} distribution range, and a second viral load in the concurrent detection case for the target virus may be calculated based on the representative value of the second C_{T} distribution range. For example, the first viral load for PIV1 may be calculated as a value inversely proportional to the median value (e.g., 20) of the first C_{T} distribution range for PIV1, and the second viral load for PIV1 may be calculated as a value inversely proportional to the median value (e.g., 32) of the second C_{T} distribution range for PIV1.

In an embodiment, when the difference between the first viral load in the single detection case and the second viral load in the concurrent detection case is not less than a predetermined reference value, the recommendation information providing unit 340 may provide a message that providing a medical treatment service in consideration of the difference is recommended. For example, as shown in reference number 30 of FIG. 4, if the C_{T} value in the concurrent detection case is greater than that in the single detection case for the same pathogen, it means that the viral load of the corresponding pathogen in the human body may have been relatively lowered by other pathogens that are concurrently infected at that time. Accordingly, the recommendation information providing unit 340 may calculate the first viral load and the second viral load of the corresponding pathogen, and when the difference in the viral loads is relatively large, control the output of a message recommending the provision of a medical treatment service with a relatively weak intensity.

In an embodiment, when the above-described C_{T} distribution range is displayed, the recommendation information providing unit 340 may perform control such that the first viral load and the second viral load for the corresponding target virus and the message are displayed together on the corresponding screen. Alternatively, the recommendation information providing unit 340 may calculate the first viral load and the second viral load in response to a user input requesting provision of the viral load, and may control that the viral loads are displayed together with the message on the sixth screen.

In an embodiment, the recommendation information providing unit 340 may receive information about a scheduled target analyte from the user terminal 200. Here, the scheduled target analyte refers to a target analyte for which a diagnostic test is scheduled for a specific patient. The recommendation information providing unit 340 may control that a message is output, which is recommending that any one of (a) the singleplex diagnostic product for detecting the presence or absence of only the scheduled target analyte among the plurality of target analytes and (b) the multiplex NAAT panel or the multiplex molecular diagnostic product for detecting the presence or absence of another target analyte together with the presence or absence of the scheduled target analyte is used to a specific patient, by using the scheduled target analyte and the count of examinees described above. For example, when the scheduled target analyte is AdV, the recommendation information providing unit 340 may generate a message recommending the use of the singleplex molecular diagnostic product targeting only AdV or the multiplex molecular diagnostic product targeting both AdV and another target analyte, by using the difference between the count of the first examinees and the count of the second examinees for AdV.

In an embodiment, the recommendation message may include a message recommending the use of the multiplex diagnostic product when the difference between the count of the first examinees and the second examinees for the scheduled target analyte satisfies a predetermined third condition. In an embodiment, the third condition may include: (a) a condition in which a ratio of the count of the second examinees to the count of the first examinees is not less than a predetermined value; (b) a condition in which the count of the second examinees is not less than a predetermined value; and (c) a condition in which a value obtained by subtracting the count of the first examinees from the count of the second examinees is not less than a predetermined value, for the scheduled target analyte. For example, the recommendation message may further include a first message recommending the use of the singleplex molecular diagnostic product when the ratio of the count of the second examinees to the count of the first examinees for AdV is less than a predetermined reference value, and may further include a second message recommending the use of the multiplex molecular diagnostic product when the corresponding ratio is equal to or greater than the reference value.

In an embodiment, the recommendation information providing unit 340 may receive a patient data about the specific patient from the user terminal 200. For example, the patient data may include data about at least one of an area and an age of the patient.

In an embodiment, the recommendation information providing unit 340 may control that the recommendation message is output, by using (a) at least one of the region and age included in the received patient data, (b) the scheduled target analyte, and (c) the count of examinees described above. For example, the use of the singleplex diagnostic product or the multiplex diagnostic product may be recommended through a process in which (a) a condition for the region and the age is determined according to the region and the age of the corresponding patient, (b) a filtered diagnostic result is obtained according to the determined condition, and (c) the count of the first examinees and the count of the second examinees for the corresponding target analyte are calculated from the filtered diagnostic result.

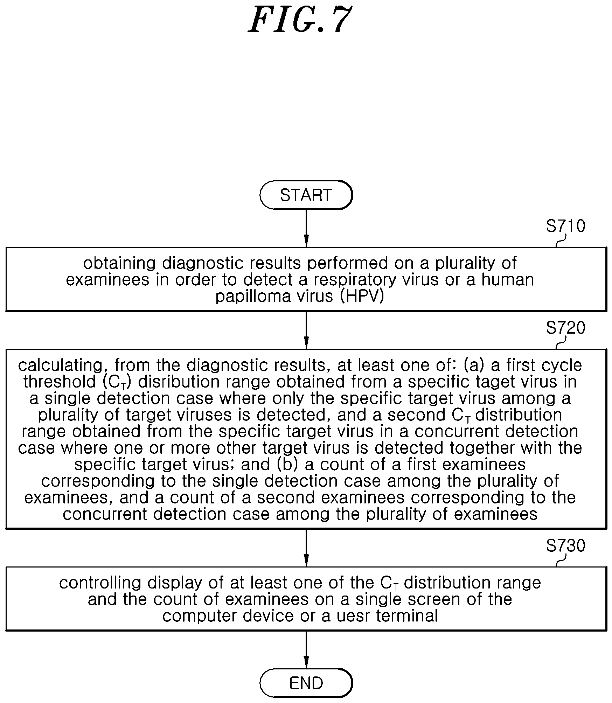

FIG. 7 is a flowchart illustrating a method for controlling display of molecular diagnostic results performed by the computer device 300 according to an embodiment.

Referring to FIG. 7, in step S710, the obtaining unit 210 may obtain diagnostic results performed on the plurality of examinees in order to detect the respiratory virus or the HPV. In an embodiment, the obtaining unit 210 may receive the diagnostic results from the database 100 or may load the diagnostic results pre-stored in the memory 220 or the storage device.

In step S720, the item value calculation unit 320 may calculate, from the diagnostic results, at least one of: (a) the first C_{T} distribution range obtained from a specific target virus in the single detection case where only the specific target virus among the plurality of target viruses is detected, and the second C_{T} distribution range obtained from the specific target virus in the concurrent detection case where one or more other target virus is detected together with the specific target virus; and (b) the count of the first examinees corresponding to the single detection case among the plurality of examinees, and the count of the second examinees corresponding to the concurrent detection case among the plurality of examinees.

In one embodiment, the item value calculation unit 320 may calculate the first C_{T} distribution range and the second C_{T} distribution range for a specific target virus from the diagnostic results. In another embodiment, the item value calculation unit 320 may calculate the count of the first examinees and the count of the second examinees for the specific target virus. In another embodiment, the item value calculation unit 320 may calculate both (a) the first C_{T} distribution range and the second C_{T} distribution range for a specific target virus, and (b) the count of the first examinees and the count of the second examinees for the specific target virus.

In an embodiment, the filtering unit 310 may filter the diagnostic results by applying the predetermined first condition for at least one of the period, the season, the region, the age, and the target virus, and the item value calculation unit 320 may calculate at least one of the count of examinees and the C_{T} distribution range from the filtered diagnostic results.

In step S730, the display control unit 330 may control display of at least one of the C_{T} distribution range and the count of examinees on a single screen of the computer device 300 or the user terminal 200.

In an embodiment, the display control unit 330 may control that the calculated first C_{T} distribution range and the calculated second C_{T} distribution range for the specific target virus are displayed on a single screen of the computer device 300 or the user terminal 200. In another embodiment, the display control unit 330 may control that the calculated count of the first examinees and the calculated count of the second examinees for the specific target virus are displayed on a single screen of the computer device 300 or the user terminal 200. In another embodiment, the display control unit 330 may control that (a) the calculated first C_{T} distribution range and the calculated second C_{T} distribution range for the specific target virus are displayed on the first screen, and (b) the calculated count of the first examinees and the second examinees for the specific target virus are displayed on the second screen or on another region within the first screen.

In an embodiment, when the computer device 300 is implemented as a computer used by a user, the display control unit 330 may transmit at least one of (a) the calculated C_{T} distribution ranges and (b) the calculated counts of examinees to the display 230, and may control the display 230 to display the at least one of (a) the C_{T} distribution ranges and (b) the counts of examinees in a single screen.

In another embodiment, when the computer device 300 is implemented as a server, the display control unit 330 may transmit at least one of (a) the calculated C_{T} distribution ranges and (b) the calculated counts of examinees to the user terminal 200, thereby displaying the at least one of (a) the C_{T} distribution ranges and (b) the counts of examinees on a single screen by user terminal 200.

According to an embodiment of the present disclosure, by providing the C_{T} distribution range in each of the single detection case and the concurrent detection case for the specific target analyte, it is possible to support a user to obtain various insight based thereon.

According to an embodiment of the present disclosure, by providing the count of examinees corresponding to each of the single detection case and the concurrent detection case for the specific target analyte, it is possible to support a user to obtain various insight based thereon.

According to an embodiment of the present disclosure, by recommending or providing the combination of the candidate target analytes for configuring the multiplex NAAT panel, the viral load of a virus(s) in a patient's body, or the viral load-based medical service, it is possible to guide a user to obtain an insight based thereon.

Combinations of each block of the block diagram attached to the present invention and each step of the flowchart may be performed by computer program instructions. Since these computer program instructions may be mounted on an encoding processor of a general-purpose computer, special-purpose computer, or other programmable data processing device, the instructions executed by the encoding processor of the computer or other programmable data processing device generate means for performing the functions described in each block of the block diagram or each step of the flowchart. Since the computer program instructions can also be stored in a computer-usable or computer-readable memory that can be directed to a computer or other programmable data processing equipment to implement functions in a particular method, the instructions stored in the computer-usable or computer-readable memory may produce an item of manufacture including instruction means for performing the functions described in each block of the block diagram or each step of the flowchart. Since the computer program instructions can also be mounted on a computer or other programmable data processing device, the instructions that perform a series of operation steps on the computer or other programmable data processing device to generate a computer-executable process, thereby performing the computer or other programmable data processing device, can also provide steps for executing the functions described in each block of the block diagram and each step of the flowchart.

In addition, each block or each step may represent a module, a segment, or a portion of code including one or more executable instructions for executing the specified logical function(s). It should also be noted that in some alternative embodiments, functions mentioned in the blocks or steps may occur out of order. For example, two blocks or steps shown in succession may in fact be performed substantially simultaneously or the blocks or steps may sometimes be performed in reverse order depending on the corresponding function.

The above description is merely illustrative of the technical idea of the present disclosure, and those skilled in the art to which the present disclosure pertains will be able to make various modifications and variations without departing from the essential quality of the present disclosure. Accordingly, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

## Claims

1. A method for controlling display of molecular diagnostic results performed by a computer device, the method comprising:
obtaining diagnostic results performed on a plurality of examinees in order to detect a respiratory virus or a human papilloma virus (HPV); wherein the respiratory virus comprises at least two types of respiratory viruses as a target virus and the HPV comprises at least two types of HPV subtypes as a target virus;
calculating, from the diagnostic results, at least one of: (a) a first cycle threshold (C_{T}) distribution range obtained from a specific target virus in a single detection case where only the specific target virus among a plurality of target viruses is detected, and a second C_{T} distribution range obtained from the specific target virus in a concurrent detection case where one or more other target virus is detected together with the specific target virus; and (b) a count of a first examinees corresponding to the single detection case among the plurality of examinees, and a count of a second examinees corresponding to the concurrent detection case among the plurality of examinees; and
controlling display of at least one of the C_{T} distribution range and the count of examinees on a single screen of the computer device or a user terminal.

2. The method of claim 1, further comprising:
filtering the diagnostic results by applying a predetermined first condition for at least one of a period, a season, a region, an age and a target virus, and a molecular diagnostic product,
wherein the diagnostic results in the calculating, is the filtered diagnostic results.

3. The method of claim 1, wherein the controlling the display of the computer device or the user terminal comprises:
providing a region corresponding to the specific target virus on the single screen, and
controlling that the first C_{T} distribution range and the second C_{T} distribution range are displayed in the region.

4. The method of claim 3, wherein the controlling the display of the computer device or the user terminal comprises:
controlling that at least one of a median value, a first quartile, a third quartile, an average value and a mode value is displayed in each of the first C_{T} distribution range and the second C_{T} distribution range.

5. The method of claim 4, wherein the controlling the display of the computer device or the user terminal comprises:
controlling that at least one of an interquartile range (IQR) indicating a difference between the first quartile and the third quartile, a minimum value calculated based on the first quartile and the IQR, and a maximum value calculated based on the third quartile and the IQR is further displayed in each of the first C_{T} distribution range and the second C_{T} distribution range.

6. The method of claim 3, wherein the controlling the display of the computer device or the user terminal comprises:
controlling that each of the first C_{T} distribution range and the second C_{T} distribution range is displayed in a box plot method.

7. The method of claim 1, wherein the controlling the display of the computer device or the user terminal comprises:
providing a region corresponding to the specific target virus on the single screen, and
controlling that the count of the first examinees and the count of the second examinees are displayed in the region.

8. The method of claim 1, wherein the controlling the display of the computer device or the user terminal comprises:
adding an identifiable mark for one or more target viruses of which a difference between the count of the first examinees and the count of the second examinees or a difference between a representative value of the first C_{T} distribution range and a representative value of the second C_{T} distribution range is not less than a predetermined reference value among the plurality of target viruses.

9. The method of claim 1, wherein the controlling the display of the computer device or the user terminal comprises:
for at least one target virus in which the count of the second examinees is not less than a predetermined reference value than the count of the first examinees among the plurality of target viruses, adding an identifiable mark when a representative value of the C_{T} distribution range is not less than the representative value of the C_{T} distribution range in the single detection case.

10. The method of claim 1, further comprising controlling display of information about one or more other target viruses detected together with the specific target virus in the concurrent detection case of the computer device or the user terminal.

11. The method of claim 10, wherein the information about the one or more other target viruses comprises: (a) a name of each of the one or more other target viruses detected together with the specific target virus; and (b) a count of examinees and/or a C_{T} distribution range in the concurrent detection case in which each of the one or more other target viruses are detected together with the specific target virus.

12. The method of claim 10, wherein the information about the one or more other target viruses comprises: (a) names of a plurality of combinations for the one or more other target viruses detected together with the specific target virus, and (b) a count of examinees and/or a C_{T} distribution range in a concurrent detection case in which each of the plurality of combinations are detected together with the specific target virus.

13. The method of claim 10, wherein the controlling the display of the information about the one or more other target viruses is performed corresponding to selection of a screen region displaying the second C_{T} distribution range or the count of the second examinees.

14. The method of claim 1, further comprising:
controlling display of information about the concurrent detection case of the specific target virus and information about the concurrent detection case of additional specific target virus on a single screen of the computer device or the user terminal.

15. The method of claim 14, further comprising:
when any one of the specific target viruses is selected among the specific target viruses, controlling display of information about a concurrent detection case of the selected any one specific target virus of the computer device or the user terminal.

16. The method of claim 1, wherein the at least two types of the respiratory viruses are selected from the group consisting of influenza virus, respiratory syncytial virus (RSV), adenovirus, enterovirus, parainfluenza virus (PIV), metapneumovirus (MPV), bocavirus, rhinovirus, and coronavirus.

17. The method of claim 1, wherein the at least two types of HPV subtypes are selected from the group consisting of HPV-16, -18, -26, -30, -31, -34, -35, -39, -45, -51, -52, -53, -56, -58, -59, -61, -66, -67, -68, -69, -70, and -73 belonging to a high-risk (HR) group.

18. The method of claim 1, wherein the diagnostic results are **characterized in that** they are diagnostic results generated by using the identical qPCR device and the identical same extraction method.

19. The method of claim 18, wherein the diagnostic results are **characterized in that** they are diagnostic results generated by using the identical qPCR device, the identical extraction method, the identical target signaling mechanism, and the identical polymerase mastermix.

20. A computer program stored on a computer-readable recording medium, wherein the computer program is programmed to perform each of steps included in the method of claim 1.

21. A computer-readable recording medium on which a computer program is stored, wherein the computer program is programmed to perform each of steps included in the method of claim 1.

22. A computer device comprising:
a memory configured to store at least one instruction; and
a processor configured to execute the one or more instructions stored in the memory, wherein the instructions, when executed by the processor, cause the processor to:
obtaining, by the processor, diagnostic results performed on a plurality of examinees in order to detect a respiratory virus or a human papilloma virus (HPV); wherein the respiratory virus comprises at least two types of respiratory viruses as a target virus and the HPV comprises at least two types of HPV subtypes as a target virus;
calculating, by the processor, from the diagnostic results, at least one of: (a) a first cycle threshold (C_{T}) distribution range obtained from a specific target virus in a single detection case where only the specific target virus among a plurality of target viruses is detected, and a second C_{T} distribution range obtained from the specific target virus in a concurrent detection case where one or more other target virus is detected together with the specific target virus; and (b) a count of a first examinees corresponding to the single detection case among the plurality of examinees, and a count of a second examinees corresponding to the concurrent detection case among the plurality of examinees; and
controlling, by the processor, display of at least one of the C_{T} distribution range and the count of examinees on a single screen of the computer device or a user terminal.
